# EUROPEAN PATENT SPECIFICATION

(11) **EP 2 114 137 B1**
(45) Date of publication and mention of the grant of the patent: **09.08.2017**
(21) Application number: 08719014.6
(22) Date of filing: 10.03.2008
(51) Int. Cl.: A01N 1/02, G01N 33/50, G01N 33/569

(54) **STABILISATION OF BIOLOGICAL CELL MARKERS**
STABILISIERUNG VON BIOLOGISCHEN ZELLMARKERN
STABILISATION DE MARQUEURS BIOLOGIQUES CELLULAIRES

(30) Priority: 08.03.2007 GB 0704488
(43) Date of publication of application: 11.11.2009
(73) Proprietor: The University of Nottingham, Nottingham Nottinghamshire NG7 2RD (GB)
(72) Inventor: MAY, Jane, Alison, Nottingham Nottinghamshire NG7 2UH (GB); HEPTINSTALL, Stanley, Nottingham Nottinghamshire NG7 2UH (GB); FOX, Susan, Carol, Nottingham Nottinghamshire NG7 2UH (GB)
(74) Representative: Atkinson, Jennifer
(86) International application number: PCT/GB2008/050169
(87) International publication number: WO 2008/107724

(56) References cited:
- WO-A-2004/017895
- US-A- 5 227 304
- I. A. HAGBERG & T. LYBERG: "Blood platelet activation evaluated by flow cytometry: optimised methods for clinical studies" PLATELETS, vol. 11, no. 3, 2000, pages 137-150, XP009111406
- U. M. HEGDE, A. BOWES & B. L. T. ROTER: "The effect of storage in different anticoagulants on platelet associated IgG" CLINICAL LABORATORY HAEMATOLOGY, vol. 6, 1984, pages 51-59, XP002512870
- DATABASE WPI Week 200679 Thomson Scientific, London, GB; AN 2006-776349 XP002512872 & KR 2006 007 497 A (SUH I B) 26 January 2006 (2006-01-26)
- K.-H. GROTEMEYER: "The platelet -reactivity test - a useful "by-product" of the blood-sampling procedure" THROMBOSIS RES., vol. 61, no. 4, 1991, pages 423-431, XP002512871

## Description

The present invention relates to biological cell markers, and in particular to compositions which stabilise the expression of cell surface markers. The invention includes methods and kits comprising such compositions for use in stabilising the expression of cell surface markers. The invention is especially concerned with compositions for use in stabilising the expression of cell markers (such as cell activation markers) present on blood cells, such as platelets. The invention extends to methods and kits for detecting platelet activation, and to kits for monitoring the efficacy of anti-thrombotic treatment regimes.

Blood cells, such as erythrocytes, leukocytes and platelets, circulate within blood vessels and are maintained in a stable flowing environment. When removed from the body, these cells are exposed to a new environment with sudden changes in pH, temperature and oxygen concentrations, as well as contact with foreign surfaces. Blood cells respond rapidly to these environmental changes, which result in platelet aggregation and the initiation of coagulation of the blood. The most important cells involved in the initiation of these haemostatic processes are the platelets. Platelets are anucleate, disc-shaped cells which, following injury, rapidly become sticky when activated, and release substances that cause them to aggregate together at the site of injury to form a platelet plug. These platelet plugs act to arrest bleeding and ultimately interact with coagulation proteins, such as fibrinogen to form a stable blood clot.

In pathological situations, platelets may become activated and form a thrombus within a blood vessel. Anti-thrombotic therapies therefore aim to reduce platelet activation sufficiently to inhibit thrombus formation, but not cause excessive bleeding. The ability to be able to easily monitor the degree of platelet activation following anti-thrombotic therapy or in certain disease states would therefore be extremely desirable. For example, it would be useful to confirm that administration of anti-thrombotic therapy was sufficient prior to performing cardiac intervention procedures, such as angioplasty or stent insertion, or to confirm that prescribed anti-thrombotic therapy was satisfactory following myocardial infarction or stroke.

Every cell has a coating of proteins which may be capable of binding, or adhering, to cell signalling molecules, or to the surface of other cells. Scientists have taken advantage of the biological uniqueness of these arrangements of surface proteins to tag or 'mark' cells. Hence, these unique patterns of expressed cell surface proteins are referred to as 'cell markers'. Some may be used for cell identification purposes, while others may be used for investigations of the activation state of cells since expression of some markers may change in response to changes occurring within the cell. In a non-activated cell, certain activation markers may be internalised or stored in granules within the cell. However, upon stimulation, they become exposed on the cell surface forming antigenic surfaces to which specific antibodies may be raised. These markers may be identified by specific antibodies conjugated to fluorochromes, which can be detected by various techniques including immunofluorescence microscopy, and flow cytometry. The identification of specific cell markers which may be expressed either as constitutive or inducible markers have been central to advances in many scientific fields including stem cell biology, immunology, haematology, oncology and pathology.

Previously, many cell surface markers were catalogued under the term "cluster designation marker" or "CD marker", and hundreds of markers are now listed in this way. There are many other cell markers known by names and acronyms which link them to their cell type or function. Examples include markers on blood cells, stem cells, cancer cells, and endothelial cells. For each type of cell, there are many known cell surface markers that are expressed constitutively which may act as identification markers. However, other markers which are inducible may be used to monitor cellular changes, for example in the activation state or the maturity of a cell.

Haematopoietic cells express various recognisable antigens on their surface. Some of these are expressed constitutively, while others are expressed upon activation of the cells. These antigens may be glycoproteins or integrins, and some are expressed exclusively on particular types of haematopoietic cells. Hence, they may be used to identify a specific cell type. All of these antigens have been identified as cluster-designated (CD) antigens through the use of monoclonal antibodies. For example, platelets exclusively express CD42a or CD61 on their surface when they are in both the activated and the non-activated state. In addition, cell surface markers released upon activation from either alpha or dense granules within the platelet are also identifiable as CD antigens. One of the most rapid of these responses is the expression of P-selectin (also known as CD62P) on the surface of the platelet. In a non-activated platelet, P-selectin is normally hidden within the platelet's internal alpha granule. However, P-selectin is expressed and becomes rapidly exposed on the platelet surface when the platelet is activated. Expression of P-selectin is therefore a useful early marker of platelet activation, and would provide a useful marker in a platelet activation test.

For analysing blood platelets, antibodies that are specific to platelet-expressed cluster-designated antigens, such as CD61 or CD62P, may be tagged with fluorochromes, in order to allow the biochemical properties of the platelet to be measured on a flow cytometer. These fluorescent probes are used in a variety of applications including identifying and quantifying distinct populations of cells and quantifying the degree of expression of different proteins by flow cytometry. By using fluorochromes fluorescing at different wavelengths, it is possible to analyse several parameters simultaneously. It is therefore possible to pick out expression of an activation marker, such as CD62P, as well as identify the cell type on which it is being expressed at the same time.

When blood cells are removed from the body and exposed to foreign surfaces, it is necessary to add agents to the blood to preserve their structure and function as well as the antigenic makeup of the surface of the cells. Traditionally, anticoagulants were developed to prevent blood from clotting in order to perform, for example, the microscopic examination of blood cells. Most anticoagulants work through inhibition of enzymes involved in the coagulation process. This has been commonly accomplished through the removal of calcium ions using ethylenediaminetetraacetic acid (EDTA), or sodium citrate. Other inhibitors of coagulation enzymes have also been used such as sodium fluoride, hirudin or heparin. While these substances act to maintain the blood in a liquid state and prevent clotting, they also affect other aspects of cell activation such as shape change, granule release and antigen exposure to a varying degree and will not necessarily preserve the level of antigen (cell surface marker) expression. Anticoagulant use therefore requires careful monitoring and an understanding of the role each type of anticoagulant may play in cell activation and the expression of antigens such as the activation marker P-selectin. For example, the inhibitory effects of pharmaceutical agents on platelets may be monitored more successfully in some anticoagulants than others.

In order to assess the effects of platelet stimulants on expression of the platelet activation marker CD62P, it is necessary to treat the blood samples to avoid cell-cell contact and thus prevent platelet aggregation from taking place. When platelet stimulants such as adenosine diphosphate (ADP), thrombin receptor activating peptide (TRAP), the thromboxane mimetic U46619 or collagen are added to blood, the platelets become activated and express glycoprotein IIb-IIIa on their surface. If the platelets are in close contact, the glycoprotein IIb-IIIa sites on the platelet surface link the platelets together via a fibrinogen bridge to form platelet microaggregates and, if agitated sufficiently, full aggregation will occur. It is therefore necessary to avoid excessive agitation and cell-cell contact following the addition of platelet stimulants.

Various substances may be added to the blood to preserve pH which may also affect the degree of exposure of antigens and the degree of platelet activation. It is also important to maintain the osmolarity of blood since any changes may result in rupture of cell membranes and the spilling of cell contents. This is particularly important in red cells which contain large amounts of both ADP and ATP, substances which are known to activate platelets in whole blood. Changes in osmolarity can also lead to changes in cell size through crenation or shrinkage. Buffer solutions are therefore used to preserve pH and osmolarity and these may contain a variety of substances including proteins and salts.

Further substances such as glutaraldehyde and paraformaldehyde or alcohols and tannins may be added to blood to act as fixatives. These solutions react with proteins within the cells and cross-link the proteins to retain their structure. These changes are irreversible and are similar to the tanning process. While these processes preserve cell structures, they also cause some denaturing and may therefore have a tendency to alter the expression of some antigenic markers on the surface of the cells. Some antigens may therefore be destroyed by this process while others may remain antigenic. Experience and a significant degree of understanding of the changes incurred through the use of any fixative is therefore essential in order to judge its effects on any antigen expression on platelets or other blood cells and on any fluorescent probes used.

Fixative solutions, such as formaldehyde, have been used previously in an attempt to preserve samples for flow cytometry, and much research has been conducted in an attempt to preserve samples for later analysis. However, no fixative solution has been found that is suitable for long-term storage. Furthermore, in some cases, the fixative solution has been shown to cause platelet activation itself, which is clearly undesirable. US 6,913,932 discloses a non-lytic platelet cell stabilisation composition which includes a phosphatase inhibitor, and a protease inhibitor, and reagents that generate formaldehyde-ammonium complexes with the platelets. The authors assert that their stabilisation composition provides stability for at least 24 hours, and that platelets stabilised using this composition may be used for the measurement of platelet CD62P expression. Clearly, there are many situations in which it would be desirable to stabilise platelets for much longer than 24 hours in order to provide more time for the transportation of a blood sample from the site at which the sample was taken from a subject to a diagnostic lab where the sample may be analysed. In some cases, it may be required to store a blood sample and hence stabilise the platelets for as much as 9 days or more. Accordingly, in such cases, the composition disclosed in US 6,913,932 does not stabilise platelets for sufficient time.

Hagberg and Lyberg, Platelets, vol. 11, no. 3, 2000, pages 137-150 relates to blood platelet activation evaluated by flow cytometry: optimised methods for clinical studies.

WO 2004/017895 relates to compositions and methods to preserve platelet activity after blood withdrawal from a donor.

Hedge et al, Clinical Laboratory Haematology, vol. 6, 1984, pages 51-59 relates to the use of an anticoagulant system comprising EDTA, Paraformaldehyde and PBS buffer to obtain platelets from whole blood.

It is therefore an object of the present invention to overcome or mitigate one or more of the problems of the prior art, whether identified herein or elsewhere, and to provide improved compositions which are capable of stabilising the expression of cell surface markers for extended periods of time. This would enable cell samples to be taken from a subject and transported significant distances, eg to foreign laboratories for analysis.

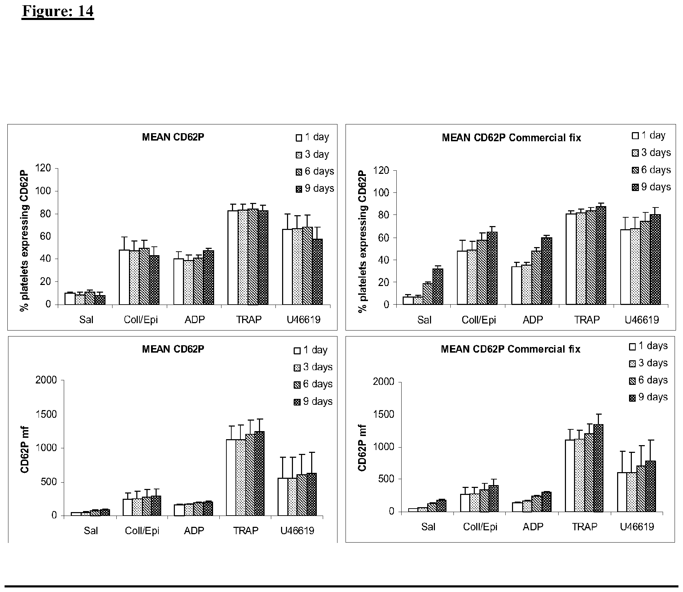

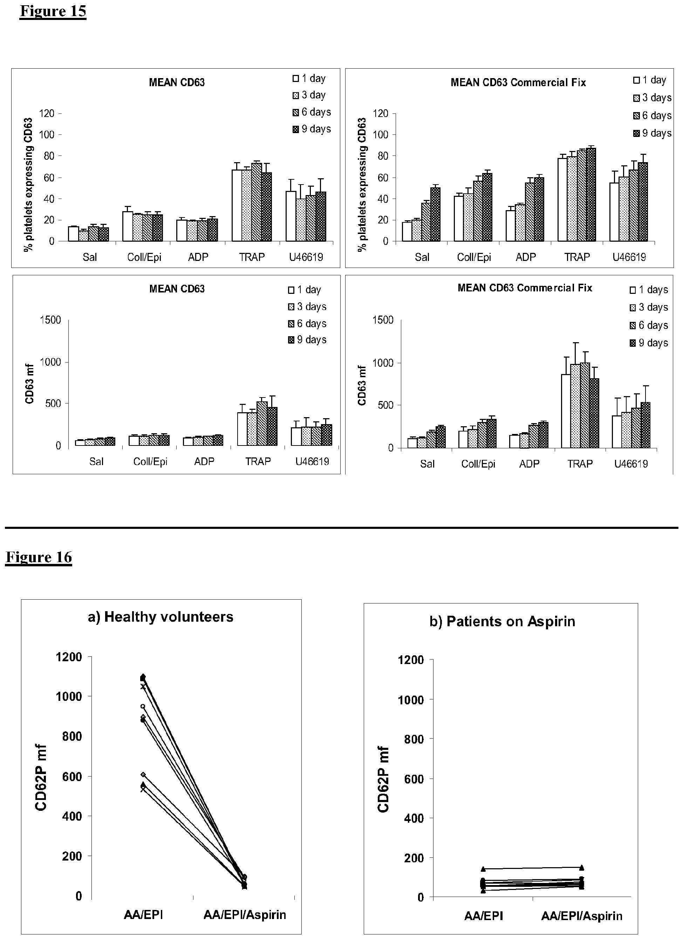

The inventors of the present invention have devised a novel cell surface marker stabilisation composition, the formulation of one embodiment of which is provided in the Examples. The composition is intended to be used to stabilise cell surface markers for cell identification purposes (for example CD61 on platelets), and in particular, to stabilise cell surface activation markers following stimulation of the cells (for example CD62P on platelets). The inventors compared the efficacy of their composition to stabilise basal levels of CD62P on platelets with that of a commercially available composition that is disclosed in US 6,913,932 (referred to under the trade name ThromboFix). No information is given in that document of the ability of Thrombofix to stabilise the high levels of expression of the platelet activation marker CD62P by its addition following stimulation of the cells. Accordingly, the inventors carried out experiments to assess and compare the efficacy of their composition with ThromboFix, and the results are shown in Figures 14 and 15.

The inventors observed that the commercially available composition was much less efficient at stabilising the platelet activation markers, CD62P and CD63. This may be deduced by the fact that the baseline level of CD62P and CD63 expression increased significantly over the 9 day period of the experiment for the saline control, and also for each of the platelet activators tested. However, in contrast, for samples stabilised with the composition devised by the inventors, baseline CD62P and CD63 expression levels remained substantially constant over the 9 day test period. Accordingly, the inventors have demonstrated that their composition is superior to the prior art composition for stabilising platelet activation markers, such as CD62P or CD63. Although they do not wish to be bound by any hypothesis, the inventors believe that the inferior stabilising effect exhibited by the prior art composition may be because the known composition contains ammonium salts which generate multiple species of formaldehyde-ammonium complexes. The inventors believe that these complexes may somehow influence the expression of the cell surface markers in some unknown mechanism such that they are not adequately stabilised.

Although the inventors were initially concerned with stabilising platelet surface markers and activation markers, they believe that their composition may be used to stabilise any cell surface markers on any cell, and that the time period by when the expression of such markers may be analysed may be significantly extended.

Hence, according to a first aspect of the invention, there is provided a cell surface marker stabilisation composition for stabilising the expression of a cell surface marker, the composition comprising: (i) an aliphatic aldehyde, (ii) a chelating agent, and (iii) a buffer, wherein the composition does not contain ammonium salt, wherein the aliphatic aldehyde is glutaraldehyde or formaldehyde, and wherein the concentration of the aliphatic aldehyde is between 0.07 and 0.2% (v/v), wherein the chelating agent is selected from a group of chelating agents consisting of ethylenediaminetetraacetic acid (EDTA), ethyleneglycol-bis(β-aminoethylether)-*N,N,N',N'*-tetraacetic acid (EGTA), *trans*-1,2-diamino-cyclohexane-*N,N,N',N'*-tetraacetic acid (CDTA), nitriloacetic acid (NTA), Porphine, Heme, 1,2-bis(o-aminophenoxy)ethane-*N,N,N',N'*-tetraacetic acid (BAPTA), Dimercaprol, or combinations thereof, and wherein the buffer comprises at least one buffering salt selected from a group of buffering salts consisting of sodium chloride, potassium chloride, disodium hydrogen phosphate, sodium dihydrogen orthophosphate monohydrate, potassium dihydrogen orthophosphate, or combinations thereof.

Advantageously, the composition according to the first aspect is for stabilising the expression of cell surface markers for significantly longer periods of time than would be possible using prior art compositions. Accordingly, a cell sample may be taken from a test subject, exposed to the composition according to the invention, and stored for long periods of time, prior to analysis of the expression level of the cell surface markers. Hence, by using the composition according to the invention, it is possible to stabilise cell samples for transportation over long distances, if required, before analysis.

By the term "cell surface marker", we mean any molecule that is attached to the plasma membrane of a cell, and which is characteristic of that cell or specific cell type.

By the term "stabilising expression of a cell surface marker", we mean maintaining or fixing the level of expression of the cell surface marker, and hence concentration of the marker on the cell surface. The cell surface marker may be expressed (ie exposed on the cell surface) constitutively (ie constantly), or its expression may be induced, for example by the presence of a cell stimulant.

By the term "cell stimulant", we mean a compound capable of inducing cell signalling pathways such that the cell surface markers that are normally internalised or hidden within intracellular stores or granules are released therefrom, and exposed on the cell's surface.

The aliphatic aldehyde is provided in the composition of the first aspect in order to react with cell surface markers present on the cells, and cross-link these proteins to retain their structure. Accordingly, their concentration (or expression level) on the cell surface is maintained for subsequent detection, for example by flow cytometric analysis.

Preferably, the aliphatic aldehyde is glutaraldehyde (or formaldehyde (ie H₂CO). It will be appreciated that formaldehyde is a gas at room temperature, and it is usually sold as a saturated aqueous solution with a concentration of about 37% formaldehyde, often stabilised with 10-15% methanol, under the trade name Formalin. Furthermore, the skilled technician will appreciate that, in addition to H₂CO, formaldehyde may exist as the cyclic trimer trioxane, and the polymeric form, paraformaldehyde.

The concentration of the aliphatic aldehyde in the composition of the first aspect is between 0.07 and 0.2% (v/v)

The chelating agent is capable of forming complexes with metal ions, and therefore inhibits the action of enzymes, such as metalloproteases, which rely on bound metals as cofactors. Hence, the chelating agent acts to preserve the cells by preventing degradation processes initiated by enzymatic attack. In embodiments where the sample is a blood sample and the cell is a blood cell, the chelating agent may advantageously act as an anticoagulant. The chelating agent forms complexes with calcium ions present in blood plasma, and prevents blood coagulation.

Most preferably, the chelating agent is EDTA. The concentration of the chelating agent in the composition according to the invention may be between about 0.01 and 5% (w/v), preferably between about 0.05 and 3% (w/v), more preferably between about 0.1 and 1% (w/v), and most preferably between about 0.1 and 0.5% (w/v).

The composition according to the invention also comprises a buffer. The buffered solution protects the cells from lysis, and preserves their integrity for at least 24 hours. Suitably, the buffer protects the cells from lysis and preserves their integrity for at least 2 days, more suitably at least 3 days, even more suitably at least 4 days, and most suitably at least 5 days. Preferably, the buffer protects the cells from lysis and preserves their integrity for at least 6 days, more preferably at least 7 days, even more preferably at least 8 days, and most preferably at least 9 days, or more.

Furthermore, the buffer is present in order to maintain the pH of the composition, and hence cell sample, at physiologically acceptable conditions. Preferably, the pH of the composition is between about 5 and 8, more preferably between about 6 and 7.5, and most preferably between about 6.5 and 7. The buffer may be selected from a group of buffers consisting of phosphate buffered saline, isotonic saline, Tris, N-(2-acetamido)-2- iminodiaacetic acid, or buffers prepared from pyrophosphate, 4-(2- hydroxyethyl)-1-piperazineethanesulfonic acid (HEPES), acetate, imidazole, succinate, maleate, citrate, carbonate, methylethyl sulfide (MES), 3-(N-morpholino)propanesulfonic acid (MOPS), or combinations of these.

The buffer preferably comprises at least one buffering salt, and preferably a plurality of buffering salts, which maintains the composition at physiological pH. Furthermore, the inventors have found that the buffering salts do not adversely affect the cell surface marker expression stabilising function of the composition according to the invention. Preferably, the buffer comprises between about 0.05 and 5% (w/v) buffering salt, more preferably between about 0.1 and 3% (w/v), and most preferably between about 0.5 and 1% (w/v) buffering salt.

Where the buffer comprises sodium chloride, the buffer preferably comprises between about 0.05 and 10% (w/v) sodium chloride, more preferably between about 0.1 and 5% (w/v), and most preferably between about 0.5 and 1% (w/v) sodium chloride.

Where the buffer comprises potassium chloride, the buffer preferably comprises between about 0.001 and 1% (w/v) potassium chloride, more preferably between about 0.01 and 0.5% (w/v), and most preferably between about 0.01 and 0.05% (w/v) potassium chloride.

Where the buffer comprises potassium dihydrogen orthophosphate, the buffer preferably comprises between about 0.001 and 1% (w/v) potassium dihydrogen orthophosphate, more preferably between about 0.01 and 0.5% (w/v), and most preferably between about 0.01 and 0.05% (w/v) potassium dihydrogen orthophosphate.

Where the buffer comprises disodium hydrogen orthophosphate, the buffer preferably comprises between about 0.001 and 2% (w/v) disodium hydrogen orthophosphate, more preferably between about 0.01 and 1% (w/v), and most preferably between about 0.05 and 0.2% (w/v) disodium hydrogen orthophosphate.

Where the buffer comprises sodium dihydrogen orthophosphate monohydrate, the buffer preferably comprises between about 0.001 and 1% (w/v) sodium dihydrogen orthophosphate monohydrate, more preferably between about 0.01 and 0.5% (w/v), and most preferably between about 0.01 and 0.05% (w/v) sodium dihydrogen orthophosphate monohydrate.

The weight ratio of buffer to chelating agent may be at least 1:1, more preferably at least 2:1, even more preferably at least 3:1, and most preferably at least 5:1. The weight ratio of buffer to chelating agent may be less than 15:1, more preferably less than 12:1, even more preferably less than 10:1, and most preferably less than 8:1.

The weight ratio of buffer to aliphatic aldehyde may be at least 1:1, more preferably at least 3:1, even more preferably at least 5:1, and most preferably at least 6:1. The weight ratio of buffer to aliphatic aldehyde may be less than 12:1, more preferably less than 10:1, even more preferably less than 8:1, and most preferably less than 7:1.

The weight ratio of aliphatic aldehyde to chelating agent may be at least 0.1:1, more preferably at least 0.3:1, even more preferably at least 0.5:1, and most preferably at least 0.8:1. The weight ratio of aliphatic aldehyde to chelating agent may be less than 5:1, more preferably less than 3:1, even more preferably less than at least 2:1, and most preferably less than 1:1.

Preferably, the composition comprises 1 part by weight of aliphatic aldehyde, 0.1-5 parts by weight chelating agent, and 3-10 parts by weight buffer. More preferably, the composition comprises 1 part by weight of aliphatic aldehyde, 0.5-3 parts by weight chelating agent, and 5-8 parts by weight buffer.

Preferably, the aliphatic aldehyde, chelating agent, and buffer are dissolved in a suitable solvent. Preferably, the composition comprises at least 80% (v/v) solvent, more preferably between at least 90% (v/v), even more preferably at least 95% (v/v), and most preferably at least 99% (v/v) solvent. A preferred solvent is water.

The weight ratio of solvent to buffer may be at least 50:1, more preferably at least 100:1, even more preferably at least 120:1, and most preferably at least 145:1. The weight ratio of solvent to buffer may be less than 250:1, more preferably less than 200:1, even more preferably less than 180:1, and most preferably less than 160:1.

The weight ratio of solvent to chelating agent may be at least 500:1, more preferably at least 750:1, even more preferably at least 800:1, and most preferably at least 850:1. The weight ratio of solvent to chelating agent may be less than 1300:1, more preferably less than 1100:1, even more preferably less than 1000:1, and most preferably less than 900:1.

The weight ratio of solvent to aliphatic aldehyde may be at least 600:1, more preferably at least 750:1, even more preferably at least 850:1, and most preferably at least 980:1. The weight ratio of solvent to aliphatic aldehyde may be less than 1400:1, more preferably less than 1200:1, even more preferably less than 1100:1, and most preferably less than 1050:1.

Hence, it will be appreciated that one embodiment of the stabilisation composition according to the invention comprises a buffered formalin-EDTA solution. It will be appreciated that the components of the composition may be dissolved in any suitable volume of solvent, for example 100 ml, and that a suitable volume of the resultant solution may be used in accordance with the invention, as will be described hereinafter. However, the skilled technician will appreciate that different volumes of solvent may be used, provided that the components are fully dissolved, and that the volume of the resultant solution to be used to stabilise expression levels of cell surface markers will depend on its concentration. The Examples describe a preferred method for preparing one embodiment of the composition according to the invention (ie buffered formalin-EDTA solution), and optimum dilutions thereof when used to stabilise cell surface markers of blood cells.

The stabilisation composition of the invention may also comprise an anticoagulant, which may be selected from a group of anticoagulants consisting of citrate, hirudin, heparin, D-Phenylalanyl-L-propyl-L-arginine chloromethyl ketone (PPACK), EDTA, and sodium fluoride. The skilled technician will appreciate that information about anticoagulants is commonly available, and that different anticoagulants would be used at different concentrations in the composition of the first aspect. By way of example, where the anticoagulant is sodium citrate, it may be provided at a concentration of about 0.3% (w/v), whereas if the anticoagulant is hirudin, it may be provided at a concentration of about 50µg/ml.

The stabilisation composition may comprise an antioxidant. Examples of suitable antioxidants include ascorbic acid and butylated hydroxytoluene. Where the composition comprises an antioxidant, the composition preferably comprises between about 0.001 and 2 % (w/v) antioxidant, more preferably between about 0.01 and 1 % (w/v), and most preferably between about 0.05 and 0.2 % (w/v) antioxidant.

The stabilisation composition may comprise a preservative, which may be selected from a group of preservatives consisting of sodium azide, sodium benzoate and methyl hydroxybenzoate. Where the composition comprises a preservative, the composition preferably comprises between about 0.001 and 2 % (w/v) preservative, more preferably between about 0.01 and 1% (w/v), and most preferably between about 0.05 and 0.2 % (w/v) preservative.

The inventors believe that the composition according to the invention may be used to stabilise, fix or maintain the expression of cell surface markers on biological cells.

Furthermore, the inventors have developed a method for stabilising the expression of cell surface markers.

According to a second aspect, there is provided a method for stabilising a cell surface marker, the method comprising contacting a cell comprising at least one cell surface marker with the cell surface marker stabilisation composition according to the first aspect.

Preferably, the method of the second aspect comprises an initial step of obtaining, from a test subject, a sample containing a plurality of cells having cell surface markers. Suitably, the sample comprises at least 1000 cells/ml, more suitably at least 10,000 cells/ml, and even more suitably at least 100,000 cells/ml. Preferably, the sample comprises at least 1,000,000 cells/ml, more preferably at least 10,000,000 cells/ml, even more preferably at least 100,000,000 cells/ml, and most preferably at least 250,000,000 cells/ml.

Upon contacting the sample with the composition according to the first aspect, the levels of cell surface marker will become stabilised, or fixed. Hence, cell surface marker expression is stabilised at the level it would be if it had been measured immediately. Hence, the method may be used to assess the level of cell activation in samples that have been collected and stabilised for later analysis.

Preferably, the method comprises a step of contacting the cell or cell sample with a suitable cell stimulant for inducing the expression of the at least one cell surface markeron the cell, wherein the cell stimulant is selected from the group consisting of calcium ionophore A23187, N-formyl-methionyl-leucyl-phenylalanine (FMLP), a cytokine, ADP, Thrombin Receptor Activating Peptide (TRAP), U46619, Collagen, Epinephrine, Platelet Activating Factor, sodium arachidonate, ristocetin, phorbol myristate acetate (PMA), 5-hydroxytryptamine (5-HT), and sulprostone. The cell stimulant induces cell signalling pathways such that the cell surface markers that are normally internalised or hidden within intracellular stores are released therefrom, and exposed on the cell's surface.

The cell or cell sample may be contacted with the cell stimulant after the cell or sample has been contacted with the stabilisation composition of the first aspect. However, it is most preferred that the cell or cell sample is contacted with the cell stimulant before contacting with the stabilisation composition of the invention.

The cell stimulant chosen will depend on the type of cell requiring stimulation.

### Other examples of blood cell

stimulants will be known to the skilled technician. Cell stimulants may be used independently, or in combination, at an appropriate concentration of between about 0.01 pM and 1 mM.

Preferably, an aliquot of the cell sample is added to the cell stimulant. The stimulant may be pre-diluted in a volume of solvent (eg saline) so that the volume of stimulant is the same as that of the sample to be added, such that the sample is diluted by about 50%, though this ratio may be adjusted, if necessary. Alternatively, in some embodiments of the method, pre-diluted stimulant may be added to the sample instead of the sample to the stimulant. The step of contacting the cell or cell sample with the cell stimulant may be performed at room temperature (ie about 21 °C), or at an elevated temperature (eg above 25 °C, above 30 °C, or above 35 °C) for a pre-determined time, eg at least one minute, preferably at least 3 minutes, at least 5 minutes, or at least 10 minutes.

Following contacting with the cell stimulant, the method preferably comprises the step of contacting the cell or sample with the composition of the first aspect. The composition of the first aspect may be added to the stimulant-cell sample mixture after a pre-determined incubation time at a ratio of 2 parts of fixative per 1 part diluted sample, though this ratio may be adjusted, if necessary. Stabilised samples may be stored at a temperature of between about 2°C and 40°C (preferably between about 4°C and 25°C), until subsequent analysis. Samples may be transported in a cool box or on ice (to maintain ambient temperatures) to a central laboratory for analysis to take place. The sample may be stored for at least 1, 2, 3, 4, or 5 days.
Preferably, the sample may be stored for at least 6, 7, 8, 9, or 10 days or more.

In some embodiments of the method, the degree of sample dilution or the ratio of diluted cells to stabilisation composition may need to be varied as necessary. The type and amount of stimulant added to the sample prior to the stabilisation composition may also be varied according to the requirements of the test.

Additionally, the method may comprise a step of contacting the cell or cell sample with substances other than a cell stimulant. For example, substances such as known cell inhibitors or cell enhancers of cell activation may be added to provide further information on cell surface marker expression. A cell enhancer is a compound which, on its own, will not stimulate a cell, but in combination with a stimulant, will enhance the degree of stimulation. A cell inhibitor is a compound, which in combination with a cell stimulant, will inhibit the degree of stimulation. In some embodiments of the method of the second aspect, prior to addition of the stabilisation composition of the first aspect, it may be desirable to contact the cell or cell sample with an antibody that is capable of identifying the specific cell-type and/or a specific cell surface marker on that cell-type. However, most preferably an antibody may be added after contacting with the composition of the first aspect.

In some embodiments, the cell sample may be collected and added immediately to the stabilisation composition of the first aspect for the evaluation of cell surface marker expression in the absence of stimulation by a stimulant.

Preferably, the method comprises a step of detecting expression levels of the cell surface marker on cells in the stabilised sample. Preferably, said detection step is carried out using flow cytometry, which will be known to the skilled technician. Flow cytometry tubes are preferably prepared containing an appropriate volume of suitably diluted cell identifier molecules, such as antibodies capable of identifying the specific cell-type and a specific cell surface marker on that cell-type. For example, where the cell type is a platelet, preferably a specific platelet identifier (such as CD61-PerCP) is added to all tubes. Antibody to CD62P-FITC (P-selectin) may also be added to the tubes, except for a control tube containing the same dilution of an appropriate control, IgG-FITCr. Preferably, antibodies to CD61 and CD62P conjugated to PerCP and FITC respectively are used. However, it will be appreciated that platelet-specific antibodies other than that to CD61 may also be used, and fluorochromes other than PerCP and FITC may be substituted provided that they are found to be suitable for the analysis of fixed P-selectin samples.

A control antibody may be used to check for non-specific binding of the antibody and to set the baseline for cell surface marker expression. The stabilised cell sample may be mixed ensuring that any sedimented cells are re-suspended, and 10µl of the sample may then be added to the flow cytometry tubes containing the antibodies and incubated for 20-30 minutes at 4°C in the dark.

Following incubation, a volume of FACSflow or any other suitable flow cytometry diluent may be added to the tubes immediately prior to reading on a flow cytometer. Preferably, the flow cytometer is equipped with a laser operating at an excitation wavelength of about 488nm and with detectors for measurement at suitable wavelengths for the selected fluorochromes. Appropriate software may be used to analyse the data from dot plots and frequency histogram plots. About 3,000 platelet events may be recorded and expression of the cell surface marker may then be quantified as the percentage of fluorescent cells present in an interval gate pre-set at 1% using an appropriate IgG control. Median fluorescence values for the cell surface marker may also be measured for the whole population of CD61 positive cells.

The use of the second aspect, or the method of the second aspect, may involve the stabilisation of a cell surface marker on any type of cell. The cell surface marker may be expressed on the cell surface constitutively (ie all the time). Alternatively, cell surface marker expression may be induced by a cell activator compound. Hence, such markers are referred to as cell surface activation markers.

Hence, the cell comprising the cell surface marker may be, for example a stem cell, an endothelial cell, or a blood cell. The blood cell may be a red blood cell (ie erythrocyte), a white blood cell (ie a leukocyte), or a platelet.

An example of a stem cell surface marker that is expressed constitutively is CD34. Other markers may become expressed on the cell as it matures, and CD34 expression may be reduced. Many cells express surface markers, the expression of which may be stabilised with the composition of the first aspect.

For example, a suitable erythrocyte cell surface marker may be CD235a. However, an endothelial cell constitutively expresses the marker CD31. An example of an endothelial cell surface activation marker may be CD62E. There are many examples of leukocyte surface markers that are expressed constitutively, such as CD14 on monocytes, CD15 on neutrophils, and lymphocyte markers, such as CD4 and CD8. There are vast numbers of lymphocyte markers specific to T cells and B cells, for example the activated T-cell marker CD25. An example of a leukocyte surface activation marker may be CD11 b which is expressed on activated neutrophils and monocytes.
All of these markers are described in reference material on cluster differentiation (CD) markers and human leukocyte differentiation antigens, and will be known to the skilled technician, for example on the website: www.hlda8.org/, or in articles, such as Zola H, Swart B, Nicolson I. CD molecules 2005: Human cell differentiation molecules. Blood 2005; 106:3123-3126.

Preferably, the cell is a platelet. Examples of platelet cell surface markers that are expressed constitutively are CD61, CD41 or CD42a. Examples of platelet cell surface activation markers include CD62P (P-selectin),CD63, or PAC-1.

Hence, preferably the composition of the first aspect is for stabilizing the expression of platelet cell surface markers, and most preferably platelet activation markers, such as CD62P (P-selectin), CD63, or PAC-1.

As demonstrated in the Examples, the inventors have shown that the composition according to the first aspect is capable of stabilising the expression of P-selectin for at least 9 days. This was unexpected, and enables blood samples to be efficiently stabilised for long periods of time with high confidence (ie little or no variation in expression levels for P- selectin), before they need to be analysed.

Hence, suitably the method of the second aspect comprises an initial step of obtaining a blood sample comprising at least 1000 platelets/ml, more suitably at least 10,000, and even more suitably at least 100,000 platelets/ml. Preferably, the sample comprises at least 1,000,000 platelets/ml, more preferably at least 10,000,000 platelets/ml, even more preferably at least 100,000,000, and most preferably at least 250,000,000 platelets/ml. Upon contacting the sample with the composition of the first aspect, the levels of platelet CD62P activation marker are stabilised or fixed. Hence, the method may be used to assess the level of platelet activation in samples of blood that have been collected and fixed for later analysis. Accordingly, platelet CD62P expression will be stabilised at the level it would be if it had been measured immediately.

In embodiments of the method where the cell is a blood cell, it may be desired to prevent blood clots from forming in the blood sample, and so the method according to the second aspect may comprise a step of contacting the blood sample with an anticoagulation agent prior to contacting with the composition according to the first aspect. Accordingly, the blood may be contacted following venepuncture with a suitable anticoagulant. A suitable anticoagulant may be a solution of sodium citrate or hirudin. Once the sample has been taken, the tube in which it is contained may be mixed carefully to ensure adequate dispersal of the anticoagulant in the blood. The blood sample may be left at room temperature for a fixed time period (eg about 10 minutes, or at least 60 minutes, or any time as appropriate), or processed immediately after venepuncture.

The cell sample is preferably contacted with a suitable platelet stimulant that is capable of stimulating expression of platelet cell surface markers. Examples of suitable platelet stimulants may be selected from a group of platelet stimulants consisting of ADP, Thrombin Receptor Activating Peptide (TRAP), U46619, collagen, epinephrine, Platelet Activating Factor, sodium arachidonate, ristocetin, phorbol myristate acetate (PMA), 5-hydroxytryptamine (5-HT), and sulprostone, or any other platelet stimulant chosen. Platelet stimulants may be used independently, or in combination, at appropriate concentrations of between about 0.01 pM and 1 mM.

Hence, preferably an aliquot of anticoagulated blood is added to the stimulant. The step of contacting the blood sample with the stimulant may be performed at room temperature, or at an elevated temperature (eg 37°C) for a pre-determined time, and then fixed by contacting the blood sample with the composition according to the first aspect. Following contacting with the platelet stimulant, the method preferably comprises the step of contacting the sample with the composition of the first aspect. For blood samples, use of the composition of the invention preserves the enhanced or stimulated degree of CD62P expression obtained following addition of the platelet stimulant.

The composition of the first aspect may be added to the stimulant-blood mixture and mixed by inversion. Stabilised samples may be stored at between 4°C and 40°C, until staining and subsequent analysis. Platelet CD62P expression would be stabilised at the level it would have been if measured immediately. Preferably, the method comprises a step of determining expression levels of the platelet marker on platelets in the stabilised samples. For example, the sample may be analysed to assess the expression of P-selectin on the surface of the platelets in the sample. Preferably, said detection step is carried out using flow cytometry, which will be known to the skilled technician. Preferably, a specific platelet identifier (such as antibody to CD61-PerCP) is added to all tubes. Antibody to CD62P-FITC (P-selectin) may also be added to all tubes, except for a control tube containing the same dilution of an appropriate control, IgG-FITC. Antibodies to CD61 and CD62P conjugated to PerCP and FITC respectively are preferred. However, platelet-specific antibodies other than CD61 may also be used, and fluorochromes other than PerCP and FITC may be substituted provided that they are found to be suitable for the analysis of fixed P-selectin samples.

The inventors have devised a kit for use in stabilising cell surface markers, such as platelet activation markers.

Hence, according to a third aspect, there is provided a cell surface marker stabilisation kit, the kit comprising a cell surface marker stabilisation according to the first aspect, a container in which the cell surface marker stabilisation composition is contained, and optionally instructions for use.

The kit may comprise sample extraction means for obtaining a cell sample from a test subject. It is preferred that the kit is a blood cell surface marker stabilisation kit, for example for stabilising platelet activation markers. Hence, the sample extraction means may comprise blood extraction means, for example a needle or syringe or the like. The test subject may be a mammal, and is preferably a human.

The kit preferably comprises a sample collection container for receiving the extracted cell sample. In embodiments where the sample is a blood sample, the sample collection container may contain an anticoagulant in order to prevent the blood from clotting. Preferably, the kit comprises at least one cell activation container containing a cell stimulant, and preferably a plurality of activation containers, each containing a different cell stimulant. Examples of suitable cell stimulants include calcium ionophore A23187, FMLP, or any other cell stimulant which may be suitable. Cell stimulants may be used independently or in combination, at appropriate concentrations of between 0.01 pM and 1 mM.

In embodiments where the cell is a platelet, the cell stimulant may be a platelet stimulant, which may be selected from a group of platelet stimulants consisting of ADP, TRAP, U46619, collagen, epinephrine, Platelet Activating Factor, sodium arachidonate, ristocetin, PMA, 5-HT, and sulprostone, or any other suitable platelet stimulant.

Preferably, the kit comprises a positive control container containing a compound that is known to induce expression of cell surface markers, preferably of the cells in the sample. Preferably, the kit comprises a negative control container containing a compound which does not induce expression of cell surface markers, such as saline.

In use, an operator may obtain a cell sample (such as a blood sample) from the subject using the extraction means. The extracted cell sample may then be inserted into the collection container. In embodiments where the sample is blood, the operator may contact the blood sample with anticoagulant. The operator may then contact the cell sample with a suitable cell stimulant under conditions to stimulate the cell. The stimulant may be transferred to the sample collection container, or the sample may be transferred to one or more cell activation containers. Suitable conditions for stimulation may be at a temperature of between 21°C and 40°C. Once the cell sample has been stimulated, it is then preferably contacted with the stabilisation composition of the first aspect. Once stabilised, the collection tube containing the cell stimulant and the stabilisation composition may then be stored, and transported to a laboratory equipped to determine the level of cell activity based on the expression levels of the cell surface markers.

Many anti-thrombotic therapies are currently designed to reduce the degree of platelet activation to inhibit the formation of a thrombus. The inventors believe that the composition of the first aspect, and hence the kit of the third aspect, may be used to monitor the degree of platelet activation following anti-thrombotic therapy. As described in Example 16, the inventors have shown that the composition may be used to confirm that administration of anti-thrombotic therapy, such as application of aspirin to a patient, has been sufficient prior to performing cardiac intervention procedures, such as angioplasty or stent insertion. Alternatively, it may be used to confirm that prescribed anti-thrombotic therapy has been satisfactory following myocardial infarction or stroke. Hence, preferably the kit of the third aspect may be an anti-thrombotic treatment kit.

There is provided an anti-thrombotic treatment kit for assessing the efficacy of anti-thrombotic treatment, the kit comprising a container in which the composition according to the first aspect is contained, and optionally instructions for use.

The kit may be used for assessment of aspirin therapy. The kit preferably comprises sample extraction means for obtaining a blood sample from a subject, for example a needle or syringe. The kit preferably comprises a sample collection container for receiving the extracted blood sample. Preferably, the sample collection container contains an anticoagulant. An example of a suitable anticoagulant includes sodium citrate. Preferably, the kit comprises at least one blood cell activation container containing a blood cell stimulant, and preferably a plurality of activation containers, each containing a different blood cell stimulant. For example, the cell stimulant may be arachidonic acid or epinephrine, or a combination thereof. Preferably, the kit comprises a positive control container containing a compound that is known to induce expression of cell surface markers of the cells in the sample. The compound may be a blood cell activator compound, such as arachidonic acid/or and epinephrine, as described in Example 16. Preferably, the kit comprises a negative control container containing an anti-thrombotic compound, such as aspirin. The kit may comprise a further control container in which a combination of the blood cell activator compound and anti-thrombotic compound may be contained.

In use, the blood sample may be incubated in a cell stimulant container for an appropriate time of at least 1 minute but up to 10 minutes (or any time as appropriate). Following stimulation, the sample may be contacted with the stabilisation composition of the first aspect in order to preserve the cells to await flow cytometric analysis. Preferred methods for carrying out the flow cytometric analysis are described herein with reference to the Examples.

The inventors also envisage using the composition of the first aspect to detect the level of cellular activity.

Therefore, according to a fourth aspect, there is provided a method of detecting the activation state of a cell, the method comprising the steps of:
(i) contacting a test sample containing at least one cell with a cell stimulant that is capable of inducing expression of cell surface markers on the at least one cell;
(ii) contacting the test sample with the stabilisation composition according to the first aspect such that the expression level of the cell surface markers on the at least one cell is stabilised; and
(iii) detecting the expression level of the cell surface markers induced by the cell stimulant, and comparing the expression level with that of a reference sample which does not contain the cell stimulant, wherein a difference in the expression level of cell surface markers between the test sample and the reference sample corresponds to the activation state of the at least one cell in the test sample.

By the term "activation state", we mean the degree or level of expression of cell surface markers on a cell, which provides an indication of how activated that cell is. For example, low concentrations (ie low expression levels) of cell surface markers on a cell indicate low activation states, whereas higher concentrations of markers on the cell indicate a higher activation state

Step (i) preferably involves stimulating the cell with a suitable cell stimulant which induces expression of the cell surface marker. It will be appreciated that the degree to which the expression may be induced will be determined not only by the cell type, but also the conditions to which it has been exposed. For example, the stimulant will induce little expression of platelet activation markers in a sample obtained from a patient undergoing anti-thrombotic therapy (eg by administration of aspirin), which is known to inhibit expression of platelet activation markers.

Preferably, step (ii) comprises contacting the stimulated cell with the composition according to the invention. This step ensures that the expression level of the cell surface marker is stabilised or fixed at the stimulated level (or at a lower level if the stimulant has little or no effect on expression levels).

Preferably, step (iii) comprises detecting the expression level of the cell surface marker induced by the cell stimulant, and then comparing this detected expression level with that of a reference sample which does not contain the cell stimulant. Preferably, the detection step is carried out using flow cytometry analysis. The inventors have found that a change in the expression level of the cell surface marker compared to the reference sample corresponds to the activation state of the cell.

Preferably, the method of the fourth aspect is used for detecting the activity state of a blood cell, and most preferably a platelet. Hence, it is preferred that the stimulant may be selected from a group of stimulants consisting of ADP, TRAP, U46619, collagen, epinephrine, Platelet Activating Factor, sodium arachidonate, ristocetin, PMA, 5-HT, and sulprostone. Preferably, step (iii) comprises detecting expression of a platelet activation marker, such as CD62P (P-selectin), CD63, or PAC-1. Most preferred activation markers are CD62P or CD63, as described in the Examples. Preferably, the method comprises use of antibodies specific for the target cell surface marker, and antibodies specific for the target cell type *per se,* which acts as a control.

The present application provides a cell activation state detection apparatus for determining the activation state of a cell in a test sample, the apparatus comprising first container containing a cell stimulant that is capable of inducing expression of cell surface markers on at least one cell in a test sample, a second container containing the stabilisation composition according to first aspect, which composition is capable of stabilising the expression level of the cell surface markers, and detection means capable of detecting the expression level of the cell surface markers induced by the cell stimulant.

Preferably, the apparatus comprises a reference cell sample which does not contain the cell stimulant. Hence, a difference in the expression level of cell surface markers between the test sample (the cells of which have been stimulated) and the reference sample (the cells of which have not been stimulated) corresponds to the activation state of the at least one cell in the test sample. The detection means preferably comprises flow cytometry apparatus.

In summary, the composition, methods and kits according to the invention are preferably used for the stabilisation of blood samples in order to measure the expression of CD62P as a marker of platelet activation. In use, a blood sample is preferably stabilised using one embodiment of the composition according to the first aspect of the invention (ie buffered formalin- EDTA solution), which has been shown to provide stability for at least 9 days. The composition preserves the surface expression of CD62P and the platelet-specific marker CD61, and advantageously does not itself induce platelet activation. A blood sample may be collected and processed using the kit according to the third aspect at a clinical site, and the sample may then be fixed with the composition of the invention. Using the method of the third aspect, the composition may be added to a blood sample immediately after venepuncture to stabilise the blood and prevent any further platelet activation following removal from the body in order to determine basal levels of CD62P expression on platelets. The composition may also be used for the stabilisation of CD62P expression on platelets that have been anticoagulated, and then activated with a suitable platelet stimulant prior to fixation.

The composition according to the first aspect stabilises the cells which may then be stored for ideally between 1 and 6 days, but up to 9 days, at a temperature of between about 4°C and 25°C. The samples may be analysed, for example by flow cytometry. Analysis may be conducted at the testing site if flow cytometry facilities are available, or alternatively the samples may be transported from the clinical site by road, rail or air freight to a central laboratory for measurement of platelet activation within the 1-9 day period. The cells may be re-suspended, by repeated inversion of the fixed blood sample prior to analysis. The inventors have surprisingly found that platelet CD62P expression remains stable throughout this treatment.

As described in the Examples, using flow cytometry procedures, platelets may be identified using a fluorescent antibody to the platelet-specific antigen CD61 using CD61-PerCP. At the same time, the degree of P-selectin expression may be assessed on the platelets by incubating them with a specific fluorescent antibody to CD62P (for example CD62P-FITC), in order to assess the extent of its expression. Expression levels may be quantified on a flow cytometer both as the percentage of the platelet population expressing the FITC-labelled CD62P in comparison with expression of a control FITC-labelled IgG antibody (ie % gated) and/or as the total CD62P-FITC fluorescence expressed by the platelet population (ie median fluorescence).

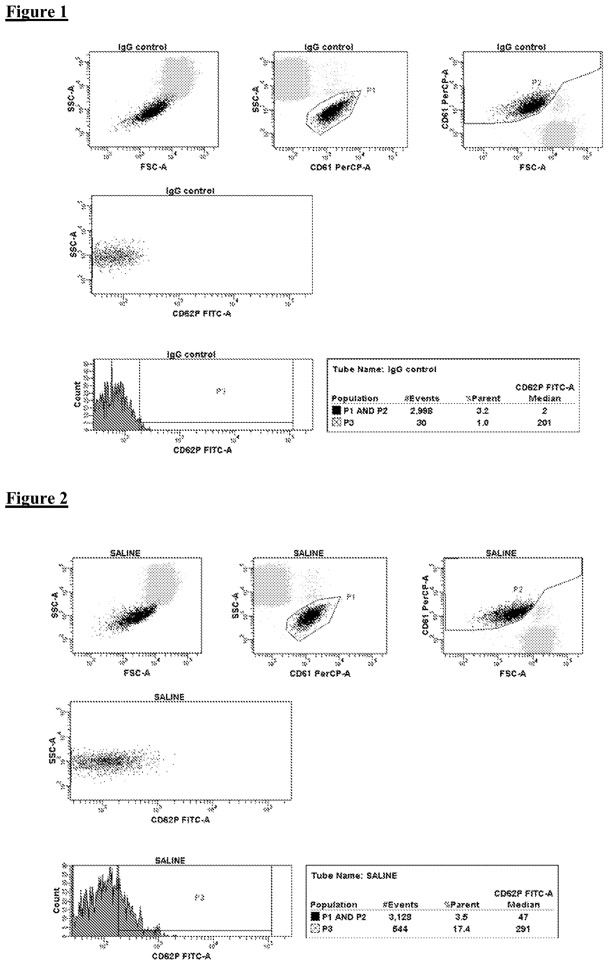

For a better understanding of the invention, and to show how embodiments of the same may be carried into effect, reference will now be made, by way of example, to the following Examples and accompanying Figures, in which:-
Figure 1 shows the results of flow cytometry analysis of platelets present in an IgG control sample, for the detection of platelet activation marker, CD62P (ie P-selectin). Top Left: is a dot plot of forward scatter (FSC) plotted against side scatter (FSC); Top Centre: is a dot plot of a platelet identifier antibody, CD61 PerCP plotted against side scatter (SSC) illustrating platelets gated in gate, P1; Top Right: is a dot plot of forward scatter (FSC) plotted against the platelet identifier antibody, CD61 PerCP, illustrating platelets gated in gate, P2; Centre: is a dot plot of the fluorescently labelled platelet activation marker, CD62P FITC plotted against side scatter (SSC) showing only events simultaneously collected from both P1 and P2; Bottom Left: is a frequency histogram of CD62P fluorescence (CD62P FITC) against cell count showing only events collected simultaneously in both P1 and P2; and Bottom Right: is a statistics box summarising the percentage of gated cells in P3 (% parent), and median fluorescence of CD62P FITC;
Figure 2 shows the results of flow cytometry analysis for the detection of platelet activation marker, CD62P, in an unstimulated sample of blood that has been diluted with saline, and then fixed;

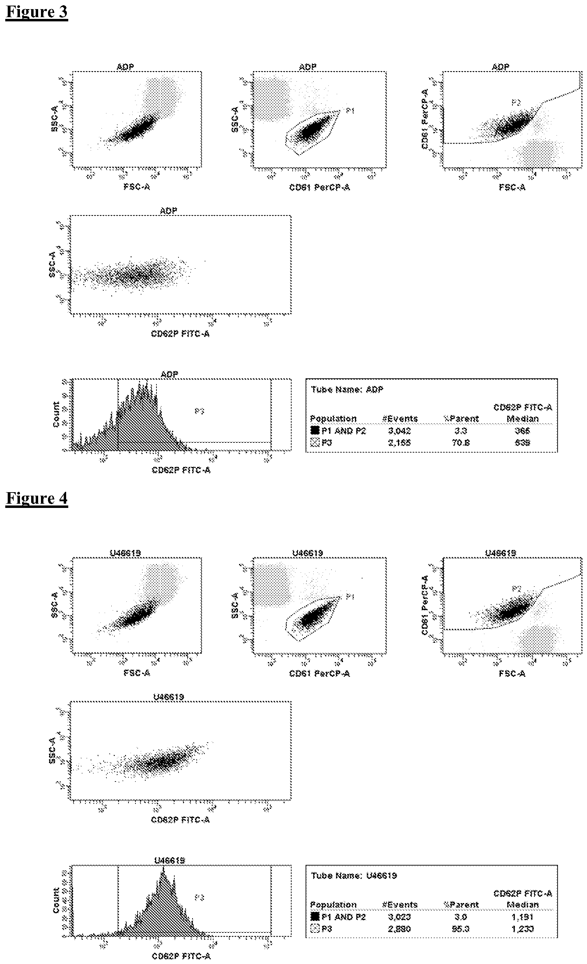

Figure 3 shows the results of flow cytometry analysis for the detection of platelet activation marker, CD62P, in a diluted sample of blood stimulated with adenosine diphosphate (ADP), and then fixed;
Figure 4 shows the results of flow cytometry analysis for the detection of platelet activation marker, CD62P, in a diluted sample of blood stimulated with a thromboxane mimetic (U46619), and then fixed;

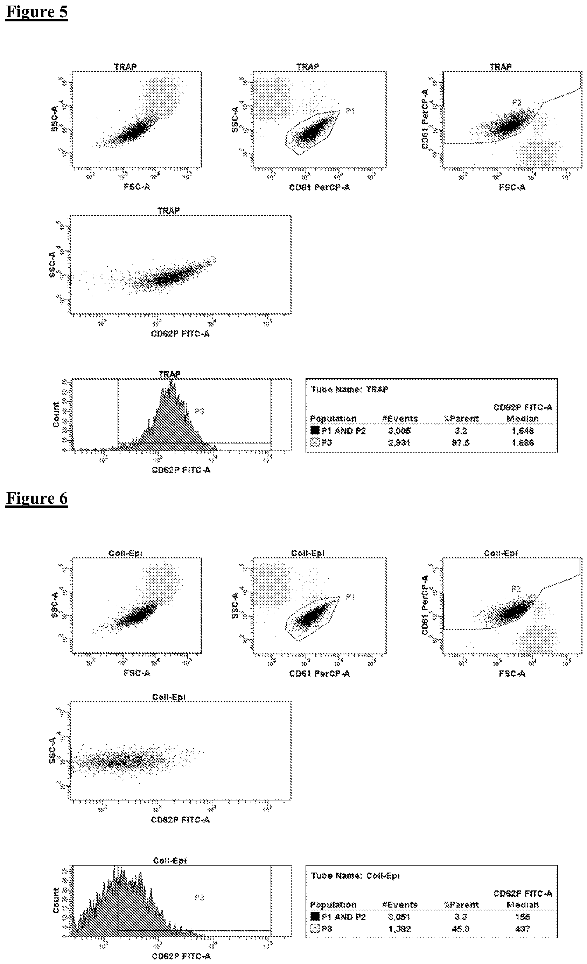

Figure 5 shows the results of flow cytometry analysis for the detection of platelet activation marker, CD62P, in a diluted sample of blood stimulated with thrombin receptor activating peptide (TRAP), and then fixed;
Figure 6 shows the results of flow cytometry analysis for the detection of platelet activation marker, CD62P, in a diluted sample of blood stimulated with a combination of collagen and epinephrine, and then fixed;

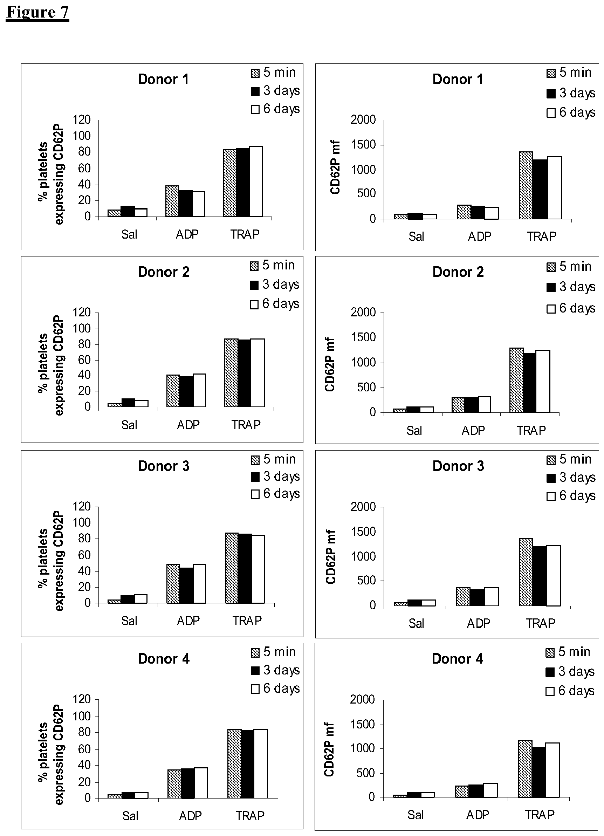

Figure 7 shows bar charts of fixed blood samples from four separate donors that had been stimulated at room temperature with platelet stimulants ADP or TRAP, or with saline alone. CD62P expression was measured within 5 minutes of fixing, and again after storage at room temperature for 3 days and 6 days. Results are shown as a percentage of platelets expressing CD62P (on the left) and median fluorescence (on the right);

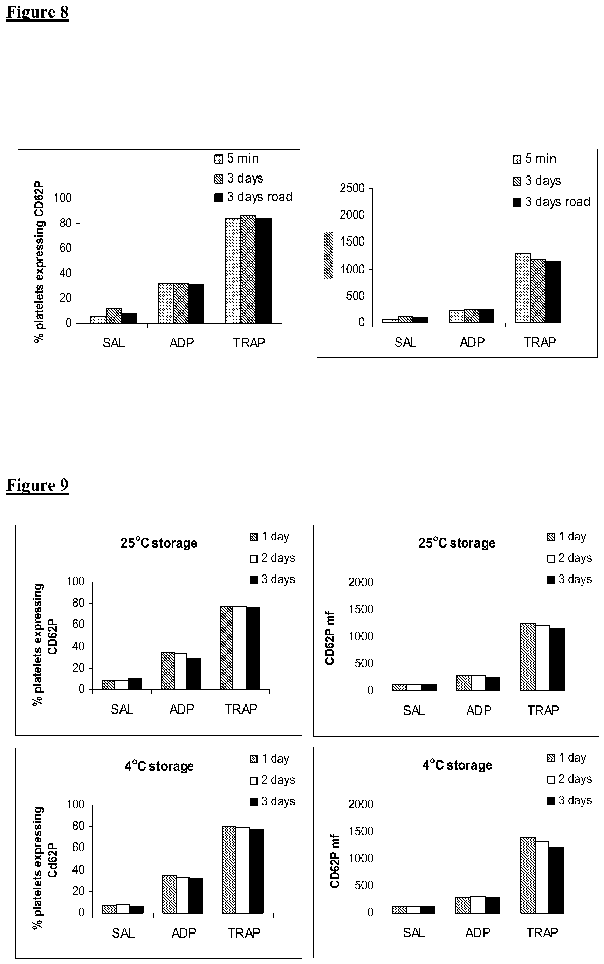

Figure 8 shows bar charts comparing the stability of CD62P expression in fixed blood samples during transportation. The blood samples had been stimulated at room temperature with platelet stimulants ADP or TRAP, or with saline alone. CD62P expression was measured within 5 minutes of fixing, and again after transportation by road for 3 days in a cool box (labelled as 3 days road) compared with 3 days storage at 4°C (labelled as 3 days). Results are shown as the percentage of platelets expressing CD62P (on the left) and median fluorescence (on the right);
Figure 9 shows bar charts comparing the stability of CD62P expression in fixed blood samples stored at 4°C and at 25°C. The blood samples had been stimulated at room temperature with platelet stimulants ADP or TRAP, or with saline alone. CD62P expression was measured after storage for 1 day, 2 days, and 3 days at either 4°C or 25°C. Results are shown as a percentage of platelets expressing CD62P (on the left) and median fluorescence (on the right);

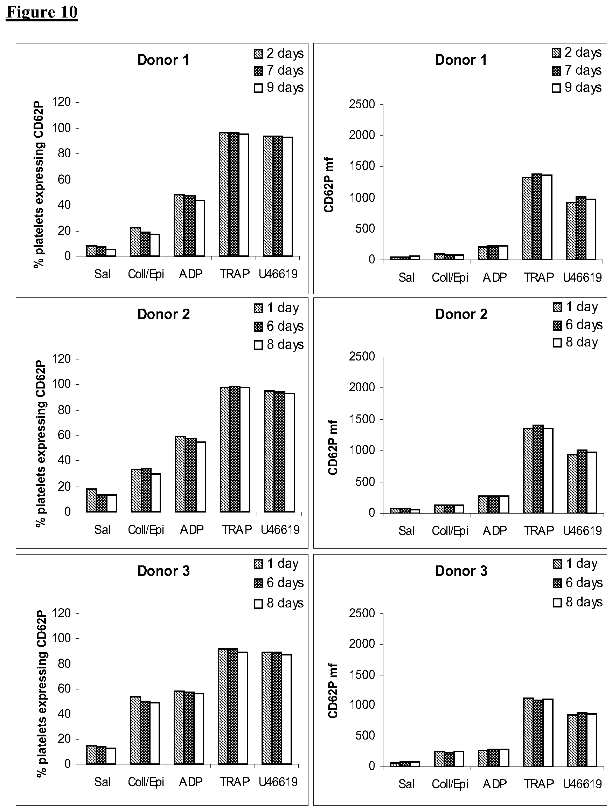

Figure 10 shows bar charts comparing the stability of CD62P expression in blood samples from three separate donors stimulated at room temperature with a range of stimulants including collagen/epinephrine, ADP, TRAP, U46619. Samples were stored at 4°C following fixing and analysed after 1-2 days, 6-7 days or 8-9 days. Results are shown as a percentage of platelets expressing CD62P (on the left) or median fluorescence (on the right);

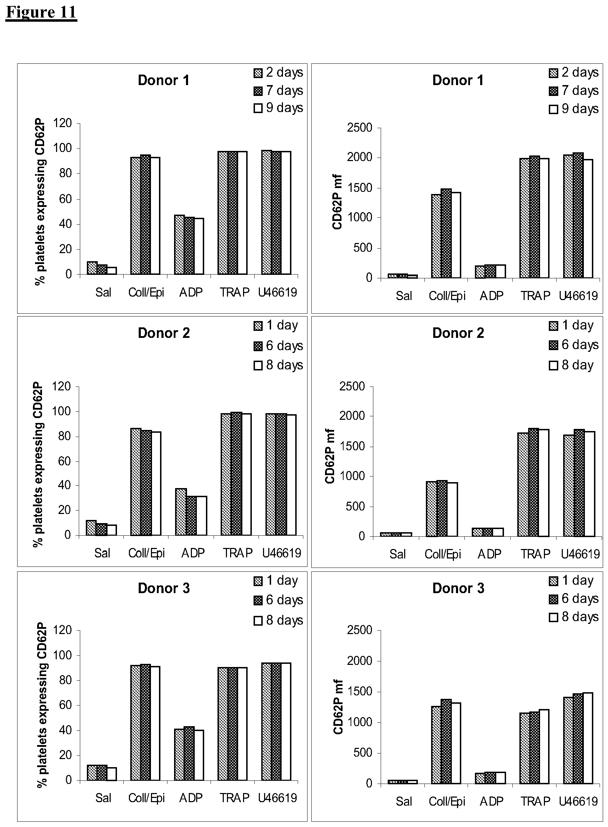

Figure 11 shows bar charts comparing the stability of CD62P expression in blood samples from three separate donors stimulated at 37°C with a range of stimulants including collagen/epinephrine, ADP, TRAP, U46619 or with saline alone. Samples were stored at 4°C following fixing and analysed after 1-2 days, 6-7 days or 8-9 days. Results are shown as a percentage of platelets expressing CD62P (on the left) or median fluorescence (on the right);

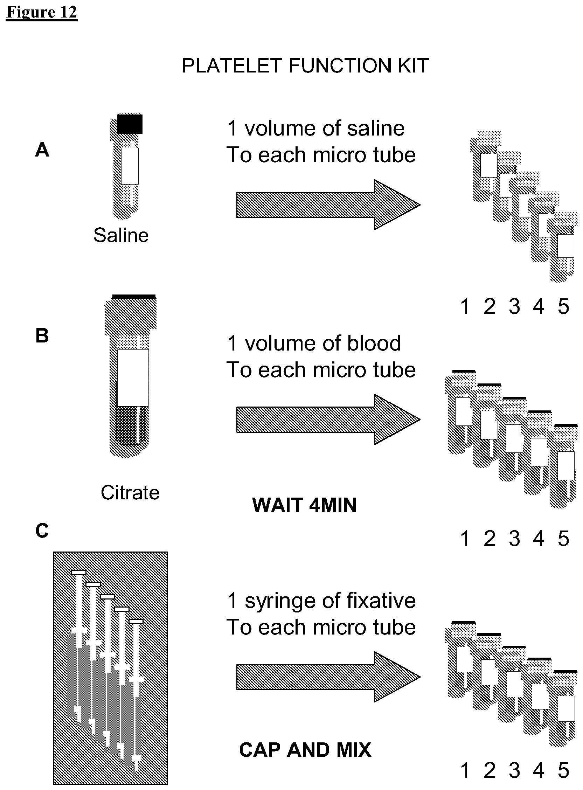

Figure 12 shows an example of a protocol sheet for a platelet activation kit according to the invention. Microtubes are pre-filled with a small volume of a platelet stimulant as required before the kit is used. This is diluted with saline at the beginning of the test (A). An equivalent volume of blood is added (B). After an appropriate time, the samples are then fixed (C) using the stabilising composition according to the invention, in preparation for transportation to a testing facility where CD62P expression may be determined;

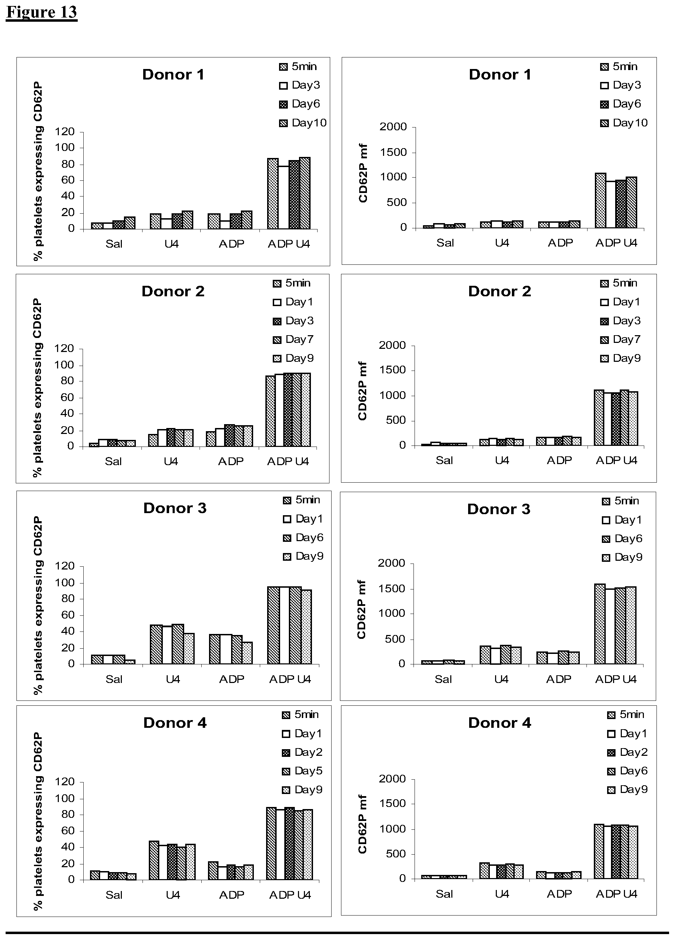

Figure 13 shows bar charts comparing the stability of CD62P expression in platelet rich plasma rather than whole blood (as shown in Figure 11) from four separate donors stimulated at 37°C with a range of stimulants including U46619 (labelled as U4 in the Figure), ADP, and ADP/U46619. Samples were stored at 4°C following fixing and analysed after 5 minutes of fixing, and again after 1, 3, 5, 6, 7, 9 or 10 days. Results are shown as a percentage of platelets expressing CD62P (on the left) or median fluorescence (on the right);
Figure 14 shows bar charts comparing the stability of CD62P expression in blood samples stabilised by the stabilisation composition according to the invention and a known commercial fixative composition, available under the trade name ThromboFix;
Figure 15 shows bar charts comparing the stability of the expression of another platelet activation marker, CD63, in blood samples stabilised by the stabilisation composition according to the invention and a known commercial fixative composition, available under the trade name ThromboFix; and
Figure 16 shows a comparison of the effect of a stimulant combination of arachidonic acid (AA) and epinephrine (EPI), or arachidonic acid (AA) and epinephrine (EPI) in the presence of aspirin (as a control) when added to blood samples obtained from a) a group of healthy volunteers who were taking no medication, and b) a group of patients on aspirin therapy. CD62P expression was measured in the blood samples between 1 and 4 days after stabilisation with the fixative composition of the invention. Results are shown as median fluorescence.

### Examples

For accurate research and blood diagnostics, it is often necessary to store and preserve blood samples in such a way that best represents the *in vivo* state of the blood sample, and not the changes caused by the removal of the sample from the body's environment. Therefore, the inventors have developed a new stabilising composition which can be used to effectively stabilise a blood sample, and particularly platelets in the blood. In particular, the inventors have found that the composition is effective at stabilising the expression of platelet activation markers, for example CD62P. Once stabilised or "fixed", the blood sample containing the platelets may be stored for a significant period of time before being examined.

The platelet stabilising composition includes: (A) a buffer solution, (B) a fixing solution, and (C) a chelating agent. The components of the platelet stabilising composition are listed in Table 1.

**Table - Ingredients of the platelet stabilising composition**

| Component | Concentration % (w/v) |
|---|---|
| | |
| (A) Buffer solution | |
| Sodium chloride | 0.8 % |
| Potassium chloride | 0.02 % |
| Potassium dihydrogen orthophosphate | 0.02 % |
| Disodium hydrogen orthophosphate | 0.141 % |
| Sodium dihydrogen orthophosphate monohydrate | 0.016 % |
| | |
| (B) Fixing solution | |
| Formaldehyde, paraformaldehyde or glutaraldehyde | 0.148 % |
| | |
| (C) Chelating agent | |
| EDTA | 0.172 % |

For 100 ml, the fixative stabiliser solution consists of an isotonic buffered component containing the five salts. Once the salts are dissolved in water, the EDTA is then added. Formalin (37% formaldehyde) is added as a 10% solution to give a final concentration of 0.4% of formalin. The quantity of 10% formalin solution is therefore 4 ml. The solution is then made up to 100ml with sterile water.

This stabiliser solution is added to blood cells at a ratio of 1 part of cells to 2 parts of stabiliser solution. This is therefore a 1 in 3 dilution, 2/3 of which is stabiliser solution according to the invention. Hence, the concentration of the ingredients in the final cells/stabiliser mixture is:

| | |
|---|---|
| Sodium chloride | 0.5333 g |
| Potassium chloride | 0.0133 g |
| Potassium dihydrogen orthophosphate | 0.0133 g |
| Disodium hydrogen orthophosphate | 0.0940 g |
| Sodium dihydrogen orthophosphate monohydrate | 0.0107 g |
| Ethylenediaminetetraacetic acid disodium salt dihydrate | 0.1146 g |
| Formalin solution | 0.2667 % |
| Formaldehyde | 0.09868 g |

The buffer solution maintains the pH value of the composition in the range of about 6-8, but ideally about pH 6.8, ie physiological pH. In addition to the three main components listed in Table 1, the platelet stabilising composition may in some embodiments also contain an anticoagulant, for example citrate, hirudin, heparin, PPACK, or sodium fluoride. In addition, in some embodiments, the composition may also include antioxidants (such as ascorbic acid), or preservatives (such as sodium azide).

The inventors then set out to test the efficacy of the platelet stabilising composition by determining the expression levels of the platelet activation marker, CD62P (ie P-selectin), using flow cytometry. Flow cytometry is a technique used for counting, examining and sorting microscopic particles suspended in a fluid stream. A beam of light is directed into a fluid stream containing the particles, and a number of detectors are aimed at the point where the stream passes through the light beam, one in line with the beam (denoted Forward Scatter or FSC) and several perpendicular to the beam (denoted Side Scatter or SSC), and one of more fluorescent detectors. Each suspended particle passing through the light beam scatters the light in a certain way, and fluorescent chemicals contained in, or attached to, the particle may be excited into emitting light at a lower frequency than the light source. This combination of scattered and fluorescent light is picked up by the detectors, and by analysing fluctuations in brightness at each detector, it is possible to extrapolate various types of information about the physical and chemical structure of each individual particle in the fluid stream.

### Example 1

Initially, an experiment was carried out in order to measure the level of expression of the platelet activation marker, CD62P, by flow cytometry in an IgG control sample. Referring to Figure 1, there are shown the results of the control experiment. Platelets were gated in both P1 (top, centre in Figure 1) and P2 (top, right in Figure 1) in plots of a platelet identifier antibody, CD61-PerCP, against both side scatter (SSC) and forward scatter (FSC). In the top left histogram SSC indicates the granularity of a cell, whereas the FSC indicates the size of the cell.

Approximately 3,000 CD61-positive platelet events were recorded. The CD61-positive cells were then drawn on a dot plot of CD62P against SSC (centre of Figure 1). A frequency histogram of CD62P fluorescence against cell count was then used to quantify the percentage of cells from the parent population that were present in an interval gate P3 (bottom, left of Figure 1). An increase in CD62P fluorescence was seen as a shift of the cell population to the right resulting in an increase in the percentage of cells present in gate P3. The gate was set at 1% using the appropriate IgG control sample and recorded in the statistics box (bottom right of Figure 1). The median fluorescence of CD62P was also recorded for the whole population of CD61 positive cells (P1 AND P2).

### Example 2

Flow cytometric analysis was carried out to determine CD62P expression in an unstimulated sample of blood diluted with saline alone and then fixed. Referring to Figure 2, there are shown the results of the experiment. As can be seen, some baseline expression of CD62P was obtained. The percentage of gated cells in P3 (% parent) is shown as 17.4, while the median fluorescence of CD62P (P1 AND P2) is 47. Hence, only a minimal increase in CD62P expression occurred upon exposure to saline alone.

### Example 3

Flow cytometric analysis was carried out to determine CD62P expression in a diluted sample of blood stimulated with adenosine diphosphate (ADP) and then fixed. The results are shown in Figure 3. Clearly, more expression of CD62P was seen following stimulation with ADP. The percentage of gated cells in P3 (% parent) had increased to from 1 to 70.8, while the median fluorescence value (P1 AND P2) had increased from 2 to 365.

### Example 4

Flow cytometric analysis was carried out to determine CD62P expression in a diluted sample of blood stimulated with the thromboxane mimetic U46619 and then fixed. The results are shown in Figure 4, and again, expression of CD62P was seen following stimulation. The percentage of gated cells in P3 (% parent) had increased from 1 to 95.3, while the median fluorescence value (P1 AND P2) was 1191.

### Example 5

Flow cytometric analysis was carried out to determine CD62P expression in a diluted sample of blood stimulated with thrombin receptor activating peptide (TRAP) and then fixed. The results are shown in Figure 5. Clearly, high expression levels of CD62P were recorded. The percentage of gated cells in P3 (% parent) was 97.5, while the median fluorescence value (P1 AND P2) was 1646.

### Example 6

Flow cytometric analysis was carried out to determine CD62P expression in a diluted sample of blood stimulated with a combination of collagen and epinephrine and then fixed. The results are shown in Figure 6, and it can be seen that a moderate level of CD62P was recorded. The percentage of gated cells in P3 (% parent) was 45.3, while the median fluorescence value (P1 AND P2) was 155.

In summary, the data generated in Example 1 to 6 demonstrate that flow cytometry may be conveniently used to monitor and quantify the expression of a platelet activation marker, such as CD62P following stabilisation with the composition of the invention.

### Example 7

The inventors wished to investigate the efficacy of their new composition at stabilising platelets in blood samples, and used CD62P as a platelet activation marker. Therefore, the stability of stabilised or fixed blood samples obtained from four separate donors that had been stimulated at room temperature with the platelet stimulants ADP or TRAP or with saline alone was then assessed. CD62P (ie P-selectin) was measured within 5 minutes of fixing (ie contacting with the composition of the invention), and then again after storage at room temperature for 3 days and then again after 6 days. The results are shown in Figure 7 as a percentage of platelets expressing CD62P (left) or median fluorescence (right). The inventors were pleased to observe that the expression of CD62P remained constant over the whole 6 days of the experiment for all donors. This is considered to be a significant advantage when using the composition of the invention for stabilising platelets. This is because someone wishing to analyse the activation level of a blood sample can be confident that testing for CD62P expression would give a true value if tested as long as 6 days after taking the sample.

### Example 8

The inventors wished to examine the stability of fixed blood samples during transportation. Blood samples were stimulated at room temperature with the platelet stimulants ADP or TRAP or with saline alone. P-selectin was then measured within 5 minutes of fixing with the platelet stabilising composition of the invention, and again after transportation by road for 3 days in a cool box compared with 3 days at 4°C. The results are shown in Figure 8 as a percentage of platelets expressing CD62P (left) or median fluorescence (right). The inventors were again pleased to see that there was little difference in the level of CD62P expression when stored in a cool box or at a fixed temperature of for 3 days.

### Example 9

The inventors then compared the stability of fixed blood samples stored at and at 25°C. Blood samples were stimulated at room temperature with the platelet stimulants ADP or TRAP or with saline alone. P-selectin was measured after storage for 1 day, 2 days and 3 days at either or 25°C. The results are shown in Figure 9 as a percentage of platelets expressing CD62P (left) or median fluorescence (right). The results show that there is no difference in CD62P activation between the samples stored at or 25°C over the 3 days tested. Hence, the inventors believe it may not be necessary to cool the samples as they are being transported to a test facility.

### Example 10

The inventors examined the stability of CD62P expression in blood samples from three separate donors stimulated at room temperature (ie about 22°C) with a range of different platelet stimulants including collagen/epinephrine, ADP, TRAP, and U46619. Samples were stored at following fixing and analysed after 1-2 days, 6-7 days or 8-9 days. The results are shown in Figure 10. The results are shown as a percentage of platelets expressing CD62P (left) or median fluorescence (right). Once again, no appreciable deviation in CD62P deviation was observed. Hence, the inventors have demonstrated that the stabilisation composition of the invention can be effectively used in combination with a wide variety of platelet stimulants if added to a blood sample at room temperature.

### Example 11

Next, the inventors wished to compare the stability of CD62P expression in blood samples from three separate donors stimulated at 37°C with the same stimulants tested in Example 10. The samples were stored at following fixing and analysed after 1-2 days, 6-7 days or 8-9 days. The results are shown in Figure 11 as a percentage of platelets expressing CD62P (left) or median fluorescence (right). As with the data of Example 10, the inventors have shown that the stabilisation composition of the invention can be effectively used in combination with a wide variety of platelet stimulants if added to a blood sample at 37°C.

### Example 12

With reference to Figure 12, the inventors have devised a method and kit for use for the anticoagulation of blood samples and subsequent fixation of the samples in order that levels of platelet CD62P are stabilised. The invention provides a method for the assessment of platelet activation in samples of blood that have been collected and fixed for later analysis. Platelet CD62P expression is stabilised at the level it would be if it had been measured immediately. The method will now be described in detail.

Blood is collected immediately into a solution of sodium citrate or hirudin, or any other suitable anticoagulant following venepuncture. Tubes are mixed carefully to ensure adequate dispersal of the anticoagulant. Blood may be left at room temperature for a fixed time period (eg 10 min), or processed immediately after venepuncture. In some circumstances, it may be desirable to store the blood at 37°C rather than at room temperature depending on which stimulant is used to activate the platelets prior to measurements of CD62P. It this case, the tubes of anticoagulated blood may be placed into a small incubator immediately after venepuncture for a pre-determined time period. In some embodiments, blood may be collected directly into the fixative/stabiliser solution in order to measure basal levels of CD62P rather than into an anticoagulant first.

Anticoagulated blood may be stimulated with a known platelet stimulant, such as ADP, TRAP, Collagen, Epinephrine, the thromboxane mimetic U46619, or any other stimulant after dilution of the blood sample. This may be performed either at room temperature or 37°C for a pre-determined time and then fixed by addition of a volume of fixative/stabiliser solution. A set of 2ml polypropylene micro tubes (or any other suitable container may be used) containing a range of platelet agonists such as ADP, Collagen, TRAP, or saline as control as appropriate is provided. An aliquot of anticoagulated blood is then added to the stimulants. The stimulants are pre-diluted in a volume of saline so that the volume of stimulant is the same as the added blood sample so that the blood is diluted by 50 %. Alternately, in some circumstances, pre-diluted stimulant may be added to the blood rather than the blood to the stimulant.

The stabilisation composition of the invention is added to the stimulant-blood mixture after a pre-determined incubation time at a ratio of 2 parts of fixative per 1 part diluted blood. The microtubes are then capped and mixed by inversion. Fixed samples are stored at between and 25°C until staining and analysis. Samples may be transported in a cool box (to maintain ambient temperatures) to a central laboratory for this analysis. Platelet CD62P expression would be stabilised at the level it would have been if measured immediately. In some situations the degree of sample dilution or the ratio of diluted cells to fixative may need to be varied as necessary. The ratios described herein are not intended to restrict the invention to these particular dilutions.

The stimulants may also be varied according to the requirements of the test and may provide a kit for analysis of a specific set of parameters. The concentrations of stimulants are pre-determined to provide suitable concentrations which would be effective in activating the platelets present in the tube. Additionally, substances other than stimulants may be added to the tubes. For example, substances such as known inhibitors or enhancers of platelet activation may be added to provide further information on P-selectin expression in these circumstances. In some circumstances, blood may be collected and added immediately to fixative for the evaluation of P-selectin in the absence of stimulation or storage.

Between 1 and 9 days following addition of fixative, the samples are then analysed to assess the expression of P-selectin on the surface of the platelets in the samples. Flow cytometry tubes are prepared containing 10µl volumes of appropriately diluted antibodies. A specific platelet identifier antibody, such as CD61-PerCP, is added to all tubes. Antibody specific to P-selectin (ie CD62P-FITC) is also added to all tubes except for a control tube containing the same dilution of an appropriate control IgG-FITC. This tube is used to check for non-specific binding of the antibody and to set the baseline for CD62P expression. The fixed blood samples are re-mixed ensuring that any sedimented red cells are re-suspended, and 10µl of the sample is added to the flow cytometry tubes containing the antibodies and incubated for 20-30 min at in the dark.

Following incubation, a volume of FACSflow or any other flow cytometry diluent is added to the tubes immediately prior to reading on a flow cytometer equipped with a laser operating at an excitation wavelength of 488nm and with detectors for measurement at suitable wavelengths for the selected fluorochromes. Appropriate software is used to analyse the data from dot plots and frequency histogram plots. 3,000 platelet events are recorded and CD62P is quantitated as the percentage of fluorescent cells present in an interval gate pre-set at 1% using an appropriate IgG control. Median fluorescence values for CD62P are also measured for the whole population of CD61 positive cells.

As described herein, antibodies to CD61 and CD62P conjugated to PerCP and FITC, respectively, are used. Platelet-specific antibodies other than CD61 may also be used, and fluorochromes other than PerCP and FITC may be substituted provided that they are found to be suitable for the analysis of fixed P-selectin samples. Appropriate assessment by an experienced flow cytometry expert of the suitability of any other platelet-specific antibody or any other fluorescent probe would be necessary before their use in the analysis of these fixed samples.

Referring to Figure 12, there is shown an example of a protocol, which may be used for a platelet activation kit in accordance with the invention. A series of microtubes (labelled 1-5) are first pre-filled with a small volume (20µl) of platelet stimulant as required before the kit is used. Examples of the platelet stimulant may be collagen/epinephrine (16mg/ml/100µM), ADP (20µM), TRAP (20µM), or U46619 (10µM).

In step (A), the volume of platelet stimulant is then diluted with 250µl of saline 0.9 % (w/v). In step (B), an equal volume (250µl) of citrate-anticoagulated blood is then added to the solution of stimulant mixed with saline. Finally, in step (C), after 4 minutes, the samples are fixed by mixing with 1 ml of platelet stabilisation composition. The resultant sample containing blood, platelet stimulant, saline, and stabilising composition may then be stored, for example at 4°C. For example, the sample may be transported from the site at which the blood sample was taken from a patient to a testing facility where the level of platelet activation may be determined. At the facility, flow cytometry analyses may be conducted to determine the expression level of platelet activation markers, such as CD62P, CD63, or PAC-1.

### Example 13

The inventors have clearly demonstrated that the stabilisation composition of the invention may be effectively used for fixing platelets in a whole blood sample, which contains erythrocytes, leucocytes, as well as platelets.
Hence, they wished to determine if the composition was equally effective for stabilising platelet rich plasma rather than whole blood. The inventors therefore compared the stability of CD62P expression in platelet rich plasma rather than whole blood from four separate donors stimulated at 37°C with a range of stimulants including U46619 (labelled as U4 in the Figure), ADP, and a combination of ADP/U46619. Samples were stored at following fixing and analysed after 5 minutes of fixing, and again after 1, 3, 5, 6, 7, 9 or 10 days. The results are shown in Figure 13 as a percentage of platelets expressing CD62P (left) or median fluorescence (right). The inventors have found that the stabilisation composition according to the invention is equally effective at stabilising platelets when present in platelet rich plasma. This is advantageous, as in many situations it may be desired to process a blood sample, for example by removing red and white blood cells, leaving blood plasma prior to determining platelet stability.

### Example 14

The inventors wished to compare the efficacy of the composition according to the present invention at stabilising platelets with that of a commercially available platelet stabilising composition referred to as ThromboFix (Beckman Coulter). Accordingly, the inventors first activated a series of blood samples at 37°C with a range of different platelet activators including collagen/epinephrine, ADP, TRAP, and U46619. Then, each solution was divided into two samples, one of which was mixed with the platelet stabilising composition according to the invention and the other of which was mixed with the commercially available platelet stabilising composition. The samples were then left for 9 days, with CD62P expression being measured at 1 day, 3 days, 6 days, and 9 day periods.

The results are shown in Figure 14. The results of stabilisation with the composition of the invention are shown on the left, and with the commercially available composition on the right. The results are shown as a percentage of platelets expressing CD62P (upper box) or median fluorescence (lower box). Comparing the percentage of platelets expressing CD62P between the two stabilising compositions, it is clear that the commercially available composition is less efficient at stabilising platelets. This may be deduced by the fact that for the saline control, and for each of the platelet activators tested, the level of CD62P expression increasing significantly with time, ie over the 9 day period of the experiment. However, in contrast, for samples stabilised with the composition according to the invention, CD62P expression levels remain constant over the 9 day period. Accordingly, the inventors have demonstrated that the composition of the invention is superior at stabilising platelet activation than the prior art composition.

### Example 15

In the preceding examples, the inventors assessed platelet activation by determining expression levels of the marker, CD62P. However, they wished to demonstrate that the composition of the invention is equally useful for stabilising platelets when investigating the expression levels of another platelet activation marker, such as CD63. Hence, using methodology similar to that of Example 14, the inventors compared the stabilising solution of the invention with the commercially available solution, Thrombofix. Blood samples from three donors were stimulated at 37°C with a range of stimulants including collagen/epinephrine, ADP, TRAP, U46619 or with saline alone (mean ± SEM). Samples were stored at following fixing and analysed after 1, 3, 6, and 9 days. The results are shown in Figure 15, as a percentage of platelets expressing CD62P (above) or median fluorescence (below).

Clearly, the data demonstrate that the composition of the invention may be efficiently and accurately used when detecting for CD63. Furthermore, the data show that CD63 expression levels remain constant over the test period, whereas they increase when the blood sample was fixed by the commercially available fixative solution.

### Example 16

The inventors have developed a kit which may be used to assess the anti-thrombotic efficacy of an anti-thrombotic agent, such as aspirin which prevents platelet activation. Blood samples were obtained from (a) a group of healthy volunteers who were not being treated with aspirin, and (b) a group of patients who were on aspirin therapy. A CD62P stimulant combination of arachidonic acid and epinephrine, or arachidonic acid and epinephrine, in the presence of aspirin (as a control), were added to each blood sample for 4 minutes, followed by the stabilisation composition of the invention. Once each sample had been stabilised in the usual manner, CD62P expression was then measured between 1 and 4 days after stabilisation, and the results are shown in Figure 16.

In the group of healthy individuals who were not being treated with aspirin, when the stimulant combination of arachidonic acid and epinephrine was administered, expression levels of the platelet activation marker CD62P were increased to between 500-1100 median fluorescence. However, for healthy blood samples that had been treated with the control stimulant containing aspirin, it can be seen that platelet activation did not occur. For the patient group on aspirin, both the blood sample combined with the stimulant arachidonic acid and epinephrine, and the control including additional aspirin, did not exhibit any platelet activation confirming that the aspirin therapy was working effectively in the patients.

Hence, it can be seen from Figure 16 that CD62P expression was completely inhibited in both groups where aspirin was added, demonstrating for group (b) that anti-thrombotic therapy was working. This demonstrates that the composition of the invention may be used to stabilise activated blood samples for subsequent detection of the platelet activation marker CD62P in a kit designed to assess the anti-thrombotic efficacy of an anti-thrombotic agent.

## Claims

1. A cell surface marker stabilisation composition for stabilising the expression of a cell surface marker, the composition comprising: (i) an aliphatic aldehyde, (ii) a chelating agent, and (iii) a buffer, wherein the composition does not contain ammonium salt, wherein the aliphatic aldehyde is glutaraldehyde or formaldehyde, and wherein the concentration of the aliphatic aldehyde is between 0.07 and 0.2% (v/v), wherein the chelating agent is selected from a group of chelating agents consisting of ethylenediaminetetraacetic acid (EDTA), ethyleneglycol-bis(β-aminoethylether)-*N,N,N',N'*-tetraacetic acid (EGTA), *trans*-1,2-diamino-cyclohexane-N,N,N',N'-tetraacetic acid (CDTA), nitriloacetic acid (NTA), Porphine, Heme, 1,2-bis(o-aminophenoxy)ethane-*N,N,N',N'*-tetraacetic acid (BAPTA), Dimercaprol, or combinations thereof, and wherein the buffer comprises at least one buffering salt selected from a group of buffering salts consisting of sodium chloride, potassium chloride, disodium hydrogen phosphate, sodium dihydrogen orthophosphate monohydrate, potassium dihydrogen orthophosphate, or combinations thereof.

2. A composition according to claim 1, wherein the concentration of the chelating agent is between about 0.01 and 5% (w/v), and preferably between about 0.1 and 0.5% (w/v).

3. A composition according to any preceding claim, wherein the composition has a pH value of between about 6 and 7.5, and preferably between about 6.5 and 7.

4. A composition according to any preceding claim, wherein the buffer comprises between about 0.05 and 5% (w/v) buffering salt, and preferably between about 0.5 and 1% (w/v) buffering salt.

5. A composition according to any preceding claim, wherein the stabilisation composition comprises an anticoagulant selected from a group of anticoagulants consisting of citrate, hirudin, heparin, D-Phenylalanyl-L-prolyl-L-arginine chloromethyl ketone (PPACK), EDTA, and sodium fluoride.

6. A method for stabilising a cell surface marker, the method comprising contacting a cell comprising at least one cell surface marker with the cell surface marker stabilisation composition according to any one of Claims 1 to 5.

7. A method according to Claim 6, wherein the method comprises a step of contacting the cell with a suitable cell stimulant for inducing the expression of the at least one cell surface marker on the cell, wherein the cell stimulant is selected from the group consisting of calcium ionophore A23187, N-formyl-methionyl-leucyl-phenylalanine (FMLP), a cytokine, ADP, Thrombin Receptor Activating Peptide (TRAP), U46619, Collagen, Epinephrine, Platelet Activating Factor, sodium arachidonate, ristocetin, phorbol myristate acetate (PMA), hydroxytryptamine (5-HT), and sulprostone.

8. A method according to Claim 7, wherein the cell stimulant is used at a concentration of between about 0.01µM and 1mM.

9. A method according to any one of Claims 6 to 8, wherein prior to contacting the cell with the stabilisation composition, the method comprises a step of contacting the cell with an antibody that is capable of identifying the specific cell-type and/or a specific cell surface marker on that cell-type.

10. A method according to any one of Claims 6 to 9, wherein the cell sample is stored for at least 1, 2, 3, 4, or 5 days, and preferably at least 6, 7, 8, 9, or 10 days or more.

11. The method according to any one of Claims 6 to 10, wherein the cell surface marker is expressed on the cell surface constitutively or induced by a cell activator compound.

12. The method according to Claim 11, wherein the cell expressing the cell surface marker is a stem cell, an endothelial cell, or a blood cell.

13. The method according to Claim 12, wherein the blood cell is a red blood cell, a white blood cell or a platelet.

14. The method according to Claim 13, wherein the blood cell is a platelet and the platelet cell surface marker is CD61, CD41, CD42a, CD62P, CD63, or PAC-1.

15. A method of detecting the activation state of a cell, the method comprising the steps of:
(i) contacting a test sample containing at least one cell with a cell stimulant that is capable of inducing expression of cell surface markers on the at least one cell;
(ii) contacting the test sample with the stabilisation composition according to first aspect such that the expression level of the cell surface markers on the at least one cell is stabilised; and
(iii) detecting the expression level of the cell surface markers induced by the cell stimulant, and comparing the expression level with that of a reference sample which does not contain the cell stimulant, wherein a difference in the expression level of cell surface markers between the test sample and the reference sample corresponds to the activation state of the at least one cell in the test sample.

16. A cell surface marker stabilisation kit, the kit comprising a cell surface marker stabilisation composition according to any one of claims 1 to 5, a container in which the cell surface marker stabilisation composition is contained and optionally instructions for use.

## Patentansprüche

1. Zelloberflächenmarker-Stabilisierungszusammensetzung zum Stabilisieren der Expression eines Zelloberflächenmarkers, wobei die Zusammensetzung umfasst: (i) ein aliphatisches Aldehyd, (ii) einen Chelatbildner und (iii) einen Puffer, wobei die Zusammensetzung kein Ammoniumsalz enthält , wobei das aliphatische Aldehyd Glutaraldehyd oder Formaldehyd ist, und wobei die Konzentration des aliphatischen Aldehyds zwischen etwa 0,07 und 0,2% (v/v) liegt, wobei der Chelatbildner ausgewählt ist aus einer Gruppe von Chelatbildnern, bestehend Ethylendiamintetraessigsäure (EDTA), Ethylenglykol-bis(β-aminoethylether)-*N,N,N',N'-*tetraessigsäure (EGTA), *trans*-1,2-Diamino-cyclohexan-N,N,N',N'-tetraessigsäure (CDTA), Nitriloessigsäure (NTA), Porphin, Häm, 1,2-Bis(o-aminophenoxy)ethan-N,N,N',N'-tetraessigsäure (BAPTA), Dimercaprol oder Kombinationen davon, und wobei der Puffer mindestens ein Puffersalz umfasst, ausgewählt aus einer Gruppe von Puffersalzen, bestehend aus Natriumchlorid, Kaliumchlorid, Dinatriumhydrogenphosphat, Natriumdihydrogenorthophosphatmonohydrat, Kaliumdihydrogenorthophosphat oder Kombinationen davon.

2. Zusammensetzung nach Anspruch 1, wobei die Konzentration des Chelatbildners zwischen etwa 0,01 und 5% (w/v) und vorzugsweise zwischen etwa 0,1 und 0,5% (w/v) liegt.

3. Zusammensetzung nach einem vorhergehenden Anspruch, wobei die Zusammensetzung einen pH-Wert zwischen etwa 6 und 7,5 und vorzugsweise zwischen etwa 6,5 und 7 aufweist.

4. Zusammensetzung nach einem vorhergehenden Anspruch, wobei der Puffer zwischen etwa 0,05 und 5% (w/v) Puffersalz und vorzugsweise zwischen etwa 0,5 und 1% (w/v) Puffersalz enthält.

5. Zusammensetzung nach einem vorhergehenden Anspruch, wobei die Stabilisierungszusammensetzung ein Antikoagulans umfasst, ausgewählt aus einer Gruppe von Antikoagulantien, bestehend aus Citrat, Hirudin, Heparin, D-Phenylalanyl-L-prolyl-L-argininchlormethylketon (PPACK), EDTA und Natriumfluorid.

6. Verfahren zur Stabilisierung eines Zelloberflächenmarkers, wobei das Verfahren das Inkontaktbringen einer Zelle, die mindestens einen Zelloberflächenmarker umfasst, mit der Zelloberflächenmarker-Stabilisierungszusammensetzung nach einem der Ansprüche 1 bis 5 umfasst.

7. Verfahren nach Anspruch 6, wobei das Verfahren einen Schritt des Inkontaktbringens der Zelle mit einem geeigneten Zellstimulans zum Induzieren der Expression des mindestens einen Zelloberflächenmarkers auf der Zelle umfasst, wobei das Zellstimulans ausgewählt ist aus der Gruppe, bestehend aus Calcium-Ionophor A23187, N-Formyl-methionyl-leucyl-phenylalanin (FMLP), einem Cytokin, ADP, Thrombinrezeptoraktivierungspeptid (TRAP), U46619, Collagen, Epinephrin, Blutplättchenaktivierungsfaktor, Natriumarachidonat, Ristocetin, Phorbolmyristatacetat (PMA), Hydroxytryptamin (5-HT) und Sulproston.

8. Verfahren nach Anspruch 7, wobei das Zellstimulans in einer Konzentration zwischen etwa 0,01 µM und 1 mM verwendet wird.

9. Verfahren nach einem der Ansprüche 6 bis 8, wobei das Verfahren vor dem Inkontaktbringen der Zelle mit der Stabilisierungszusammensetzung einen Schritt des Inkontaktbringens der Zelle mit einem Antikörper umfasst, der in der Lage ist, den spezifischen Zelltyp und/oder einen spezifischen Zelloberflächenmarker auf diesem Zelltyp zu identifizieren.

10. Verfahren nach einem der Ansprüche 6 bis 9, wobei die Zellprobe für mindestens 1, 2, 3, 4 oder 5 Tage und vorzugsweise mindestens 6, 7, 8, 9 oder 10 Tage oder mehr aufbewahrt wird.

11. Verfahren nach einem der Ansprüche 6 bis 10, wobei Zelloberflächenmarker konstitutiv auf der Zelloberfläche exprimiert wird, oder durch eine Zellaktivatorverbindung induziert wird.

12. Verfahren nach Anspruch 11, wobei die Zelle, die den Zelloberflächenmarker exprimiert, eine Stammzelle, eine Endothelzelle oder eine Blutzelle ist.

13. Verfahren nach Anspruch 12, wobei die Blutzelle eine rote Blutzelle, eine weiße Blutzelle oder ein Blutplättchen ist.

14. Verfahren nach Anspruch 13, wobei die Blutzelle ein Blutplättchen und der Blutplättchenzelloberflächenmarker CD61, CD41, CD42a, CD62P, CD63 oder PAC-1 ist.

15. Verfahren zum Nachweisen eines Aktivierungszustands einer Zelle, wobei das Verfahren die folgenden Schritte umfasst:
(i) Inkontaktbringen einer Testprobe, die mindestens eine Zelle enthält, mit einem Zellstimulans, das in der Lage ist, die Expression von Zelloberflächenmarkern auf der mindestens einen Zelle zu induzieren;
(ii) Inkontaktbringen der Testprobe mit der Stabilisierungszusammensetzung gemäß dem ersten Aspekt, so dass das Expressionsniveau der Zelloberflächenmarker auf der mindestens einen Zelle stabilisiert wird; und
(iii) Erfassen des Expressionsniveaus der durch das Zellstimulans induzierten Zelloberflächenmarker und Vergleichen des Expressionsniveaus mit dem einer Referenzprobe, die das Zellstimulans nicht enthält, wobei ein Unterschied im Expressionsniveau von Zelloberflächenmarkern zwischen der Testprobe und der Referenzprobe dem Aktivierungszustand der mindestens einen Zelle in der Testprobe entspricht.

16. Zelloberflächenmarker-Stabilisierungs-Kit, wobei der Kit eine Zelloberflächenmarker-Stabilisierungszusammensetzung nach einem der Ansprüche 1 bis 5, einen Behälter, in dem die Zelloberflächenmarker-Stabilisierungszusammensetzung enthalten ist, und gegebenenfalls Gebrauchsanweisungen umfasst.

## Revendications

1. Composition de stabilisation de marqueur de surface cellulaire pour stabiliser l'expression d'un marqueur de surface cellulaire, la composition comprenant : (i) un aldéhyde aliphatique, (ii) un agent chélateur, et (iii) un tampon, la composition ne contenant pas de sel d'ammonium, l'aldéhyde aliphatique étant le glutaraldéhyde ou le formaldéhyde, et la concentration de l'aldéhyde aliphatique étant comprise entre 0,07 et 0,2 % (v/v), l'agent chélateur étant choisi parmi a groupe d'agents chélateurs constitué de l'acide éthylènediaminetétraacétique (EDTA), l'acide éthylèneglycol-bis(β-aminoéthyléther)-*N*,*N*,*N*',*N*'-tétraacétique (EGTA), l'acide *trans*-1,2-diamino-cyclohexane-*N,N,N',N'*-tétraacétique (CDTA), l'acide nitriloacétique (NTA), la porphine, l'hème, l'acide 1,2-bis(o-aminophénoxy)éthane-*N,N,N',N'*-tétraacétique (BAPTA), le dimercaprol, ou des combinaisons de ceux-ci, et le tampon comprenant au moins un sel tampon choisi dans un groupe de sels tampons constitué des chlorure de sodium, chlorure de potassium, hydrogénophosphate disodique, dihydrogéno-orthophosphate de sodium monohydraté, dihydrogéno-orthophosphate de potassium, ou des combinaisons de ceux-ci.

2. Composition selon la revendication 1, dans laquelle la concentration de l'agent chélateur est comprise entre environ 0,01 et 5 % (m/v), et de préférence entre environ 0,1 et 0,5 % (m/v).

3. Composition selon l'une quelconque des revendications précédentes, la composition ayant une valeur de pH comprise entre environ 6 et 7,5, et de préférence entre environ 6,5 et 7.

4. Composition selon l'une quelconque des revendications précédentes, dans laquelle le tampon comprend entre environ 0,05 et 5 % (m/v) de sel tampon, et de préférence entre environ 0,5 et 1 % (m/v) de sel tampon.

5. Composition selon l'une quelconque des revendications précédentes, la composition de stabilisation comprenant un anticoagulant choisi dans un groupe d'anticoagulants constitué des citrate, hirudine, héparine, D-phénylalanyl-L-prolyl-L-arginine-chlorométhylcétone (PPACK), EDTA et fluorure de sodium.

6. Procédé de stabilisation d'un marqueur de surface cellulaire, le procédé comprenant la mise en contact d'une cellule comprenant au moins un marqueur de surface cellulaire avec la composition de stabilisation de marqueur de surface cellulaire selon l'une quelconque des revendications 1 à 5.

7. Procédé selon la revendication 6, le procédé comprenant une étape de mise en contact de la cellule avec un stimulant cellulaire adapté pour induire l'expression de l'au moins un marqueur de surface cellulaire sur la cellule, le stimulant cellulaire étant choisi dans le groupe constitué des ionophore calcique A23187, N-formyl-méthionyl-leucyl-phénylalanine (FMLP), une cytokine, ADP, peptide activateur de récepteur de thrombine (TRAP), U46619, collagène, adrénaline, facteur d'activation plaquettaire, arachidonate de sodium, ristocétine, myristate-acétate de phorbol (PMA), hydroxytryptamine (5-HT) et sulprostone.

8. Procédé selon la revendication 7, dans lequel le stimulant cellulaire est utilisé à une concentration comprise entre environ 0,01 µM et 1 mM.

9. Procédé selon l'une quelconque des revendications 6 à 8, dans lequel, avant la mise en contact de la cellule avec la composition de stabilisation, le procédé comprend une étape de mise en contact de la cellule avec un anticorps qui est capable d'identifier le type de cellule spécifique et/ou un marqueur de surface cellulaire spécifique sur ce type de cellule.

10. Procédé selon l'une quelconque des revendications 6 à 9, dans lequel l'échantillon de cellules est conservé pendant au moins 1, 2, 3, 4 ou 5 jours et, de préférence, au moins 6, 7, 8, 9 ou 10 jours ou plus.

11. Procédé selon l'une quelconque des revendications 6 à 10, dans lequel le marqueur de surface cellulaire est exprimé sur la surface cellulaire de façon constitutive ou induit par un composé activateur cellulaire.

12. Procédé selon la revendication 11, dans lequel la cellule exprimant le marqueur de surface cellulaire est une cellule souche, une cellule endothéliale ou une cellule sanguine.

13. Procédé selon la revendication 12, dans lequel la cellule sanguine est un globule rouge, un globule blanc ou une plaquette.

14. Procédé selon la revendication 13, dans lequel la cellule sanguine est une plaquette et le marqueur de surface cellulaire plaquettaire est CD61, CD41, CD42a, CD62P, CD63 ou PAC-1.

15. Procédé de détection de l'état d'activation d'une cellule, le procédé comprenant les étapes de :
(i) mise en contact d'un échantillon d'essai contenant au moins une cellule avec un stimulant cellulaire qui est capable d'induire l'expression de marqueurs de surface cellulaire sur l'au moins one cellule ;
(ii) mise en contact de l'échantillon d'essai avec la composition de stabilisation selon le premier aspect de sorte que le taux d'expression des marqueurs de surface cellulaire sur l'au moins une cellule soit stabilisé ; et
(iii) détection du taux d'expression des marqueurs de surface cellulaire induits par le stimulant cellulaire, et comparaison du taux d'expression à celui d'un échantillon de référence qui ne contient pas le stimulant cellulaire, une différence de taux d'expression des marqueurs de surface cellulaire entre l'échantillon d'essai et l'échantillon de référence correspondant à l'état d'activation de l'au moins une cellule dans l'échantillon d'essai.

16. Trousse de stabilisation de marqueur de surface cellulaire, la trousse comprenant une composition de stabilisation de marqueur de surface cellulaire selon l'une quelconque des revendications 1 à 5, un récipient dans lequel la composition de stabilisation de marqueur de surface cellulaire est contenue et, facultativement, des instructions d'utilisation.
